# EUROPEAN PATENT APPLICATION

(11) **EP 2 322 176 A1**
(43) Date of publication of application: **18.05.2011**
(21) Application number: 09382243.5
(22) Date of filing: 11.11.2009
(51) Int. Cl.: A61K 31/4375, C07D 471/04, A61P 19/00

(54) **New 7-phenyl-[1,2,4]triazolo[4,3-a]pyridin-3(2H)-one derivatives**

(71) Applicant: Almirall, S.A., 08022 Barcelona (ES)
(72) Inventor: Aiguade Bosch, José, 08980, Sant Feliu de Llobregat (ES); Carranco Moruno, Inés, 08980, Sant Feliu de Llobregat (ES)
(74) Representative: Elzaburu Marquez, Alberto

(57) **Abstract**

This invention is directed to new inhibitors of the p38 mitogen-activated protein kinase having the general formula (I), 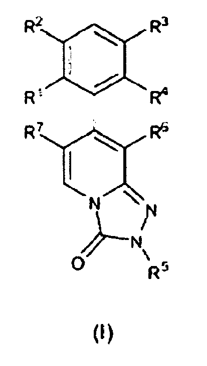 to pharmaceutical compositions comprising them, and to their use in therapy.

## Description

The present invention relates to new inhibitors of the p38 mitogen-activated protein kinase.

MAP kinases are evolutionary conserved enzymes translating membrane signals into gene expression responses. In mammals, four MAPK families can be distinguished: extracellular signal-related kinases (ERK1/2), Jun amino terminal kinases (JNK1/2/3), p38 proteins (alpha, beta, gamma and delta) and ERK5. The regulation of these proteins is exerted by a three-tier cascade composed of MAPK, MAPK kinase, and MAPK kinase kinase.

p38 MAPK was originally identified as the target of CSAIDs (cytokine suppressive antiinflammatory drugs), having a central role in the signal transduction pathway leading to the production of TNF-alpha and other cytokines (Lee et al, 1984). p38 is activated by phosphorylation in Thr and Tyr by either MKK3, MKK4, or MKK6 (Kyriakis and Avruch, 2001) in response to stress and pro-inflammatory stimuli. In turn, p38 phosphorylates its effectors in Ser and Thr residues, namely protein kinases phosphatases and transcription factors, such as ATF-2, MEF2, MAPKAPK2, MSK1/2 or MNK1/2. Altogether this activation cascade results in control of gene expression through four different mechanisms: transcription factor activation; mRNA stabilization; mRNA translation; and histone phosphorylation at NF-kB binding sites in chromatin (Shi and Gaestel, 2002; Sacanni et al, 2001).

There are four different p38 isoforms encoded by separate genes: p38 alpha, beta, gamma and delta, each one showing a distinct tissue expression pattern. As assessed by mRNA and protein levels (Beardmore et al, 2005; Wang et al, 1997), p38 alpha and beta are ubiquitously expressed, with p38 beta expression being more relevant in CNS tissues (brain, cortex, cerebellum, hippocampus, etc). The expression of p38 gamma is more prominent in skeletal muscle while p38 delta localizes mainly in heart, kidney, lung and adrenal gland. At the cellular level, p38 alpha and delta seem to be the most relevant isoforms in immune cells (monocytes, macrophages, neutrophils and T cells) (Hale et al, 1999). Pharmacological inhibition with specific p38alpha/beta inhibitors as well as gene targeting studies have indicated that p38alpha is the isoform regulating inflammatory responses most probably through its downstream substrate MAPKAP-K2 (Kotlyarov et al, 1999). Likewise, this isoform is necessary in early embryonic development as p38alpha KO (knock-out) mice die in embryonic day 12.5 due to placental insufficiency and vascular defects (Allen et al, 2000; Tamura et al, 2000; Adams et al, 2000), a phenotype that is also reproduced in the MKK3/MKK6 double KO mice (Brancho et al, 2003). In contrast, p38 beta, gamma and delta knock-out mice do not show any developmental deficiencies (Beardmore et al, 2005; Sabio et al, 2005). p38 beta KO mice appear to respond similarly to pro-inflammatory stimuli (LPS) as wild type controls, indicating that this isoform does not have a role in inflammation (Beardmore et al 2005).

The contribution of the p38MAPK pathway to inflammation has been studied both in vitro and in vivo by employing different chemical series of p38 inhibitors (Pargellis and Regan, 2003; Kumar et al, 2003). The most widely used inhibitor molecule, SB203580, is, in fact, a dual p38alpha/beta inhibitor. Inhibition of p38 abrogates the release of TNF-alpha as well as other pro-inflammatory cytokines like IL-1, IL-6, and IL-8, in PBMC, whole blood, or the human monocytic cell line THP-1.

By virtue of the involvement of p38 in TNFalpha production, inhibitors of p38 have been tested in animal models of diseases in which TNFalpha has a pathophysiological role. p38 inhibition decreases murine collagen-induced arthritis and rat adjuvant-induced arthritis severity (Pargellis and Regan, 2003). Furthermore, p38 inhibitors also improve bone resorption in animal models of arthritis, probably due to the implication of p38 MAPK in the differentiation of osteoclasts. p38 inhibition attenuates the inflammatory response in a murine model of Crohn's disease and diminishes TNF-alpha production in human Crohn's disease patient biopsies (Hollenbach et al 2005; Waetzig et al, 2002). Due to the exclusive usage of the p38 pathway by neutrophils, p38 has also been considered a target for chronic obstructive pulmonary disease (COPD) (Nick et al, 2002). p38 inhibition reduces neutrophilia, inflammatory cytokines, MMP-9 and fibrosis in lung (Underwood et al, 2000). In skin models of irradiation, inhibition of p38 protects the epidermis against acute ultraviolet radiation exposure by blocking apoptosis and inflammatory responses (Hildesheim et al, 2004). p38 inhibition also reverses hematopoietic defects in bone marrow from patients with myelodysplastic syndromes, in which TNF-alpha overproduction has a pathophysiological role (Katsoulidis et al, 2005).

In hematopoietic malignancies, a study has shown that p38 inhibitors can block the proliferation of multiple myeloma cells by inhibiting the production of IL-6 and VEGF in bone marrow stromal cells (Hideshima et al, 2002).

p38 is involved in key cellular mechanisms such as apoptosis, fibrosis and cellular hypertrophy, which are common to cardiac and vascular pathologies. Pharmacological inhibition of p38 has proven useful in improving ischemia-reperfusion injury, cerebral focal ischemia, acute coronary syndrome, chronic heart failure and post-myocardial infarction remodelling (See et al, 2004).

Experimental inhibition of p38 has been reported effective in reducing pain in animal models of neuropathy that rely on COX-2 expression and TNF-alpha production by glial cells (Schafers et al, 2003; Jin et al, 2003; Tsuda et al, 2004).

Therefore, the compounds of the invention may be useful in the prophylaxis or treatment of any disease or disorder in which p38 kinase plays a role including conditions caused by excessive or unregulated pro-inflammatory cytokine production including for example excessive or unregulated TNF, IL-1, IL-6 and IL-8 production in a human, or other mammal. The invention extends to such a use and to the use of the compounds for the manufacture of a medicament for treating such cytokine-mediated diseases or disorders. Further, the invention extends to the administration to a human an effective amount of a p38 inhibitor for treating any such disease or disorder.

Diseases or disorders in which p38 kinase plays a role either directly or via pro-inflammatory cytokines including the cytokines TNF, IL-1, IL-6 and IL-8 include without limitation autoimmune diseases, immune and inflammatory diseases, destructive bone disorders, neoplastic disorders, neurodegenerative disorders, viral diseases, infectious diseases, cardiovascular diseases, angiogenesis-related disorders, and pain-related disorders.

Autoimmune diseases which may be prevented or treated include but are not limited to rheumatoid arthritis, psoriatic arthritis, ankylosing spondilytis, Reiter's syndrome, fibromyalgia, inflammatory bowel disease such as ulcerative colitis and Crohn's disease, multiple sclerosis, diabetes, glomerulonephritis, systemic lupus erythematosus, scleroderma, chronic thyroiditis, Grave's disease, hemolytic anemia, autoimmune gastritis, autoimmune neutropenia, thrombocytopenia, autoimmune chronic active hepatitis, myasthenia gravis, or Addison's disease.

Immune and inflammatory diseases which may be prevented or treated include but are not limited to asthma, COPD, respiratory distress syndrome, acute or chronic pancreatitis, graft versus-host disease, Behcet syndrome, inflammatory eye conditions such as conjunctivitis and uveitis, psoriasis, contact dermatitis, atopic dermatitis, sarcoidosis, gout, pyresis, transplant rejection, allergic rhinitis, allergic conjunctivitis,

Cardiovascular diseases which may be prevented or treated include but are not limited to ischemia-reperfusion injury, cerebral focal ischemia, acute coronary syndrome, congestive heart failure, cardiomyopathy, myocarditis, atherosclerosis, vasculitis and restenosis.

Destructive bone disorders which may be prevented or treated include but are not limited to osteoporosis, osteoarthritis and multiple myeloma-related bone disorder.

Neoplastic disorders which may be prevented or treated include but are not limited to solid tumors such as Kaposi's sarcoma, metastatic melanoma, and hematopoietic malignancies such as acute or chronic myelogenous leukemia and multiple myeloma.

Neurodegenerative diseases which may be prevented or treated include but are not limited to Parkinson's disease, Alzheimer's disease, neurodegenerative disease caused by traumatic injury, or Huntington's disease.

Viral diseases which may be prevented or treated include but are not limited to acute hepatitis infection (including hepatitis A, hepatitis B and hepatitis C), HIV infection, Epstein-Barr infection, CMV retinitis, SARS or avian influenza A infection.

Infectious diseases which may be prevented or treated include but are not limited to sepsis, septic shock, endotoxic shock, Gram negative sepsis, toxic shock syndrome, Shigellosis, or cerebral malaria.

Angiogenesis-related disorders which may be prevented or treated include but are not limited to hemangiomas, ocular neovascularization, macular degeneration or diabetic retinopathy.

Pain-related disorders which may be prevented or treated include but are not limited to neuropathic pain (such as diabetic neuropathy, post-herpetic or trigeminal neuralgia), cancer-related pain, chronic pain (such as lower back pain syndrome), and inflammatory pain.

Other miscellaneous diseases or disorders which may be prevented or treated include but are not limited to myelodysplastic syndrome, cachexia, endometriosis, acute skin injuries such as sunburn, and wound healing.

In view of the physiological effects mediated by inhibition of the p38 mitogen-activated protein kinase, several compounds have been recently disclosed for the treatment or prevention of rheumatoid arthritis, ischemia-reperfusion injury, cerebral focal ischemia, acute coronary syndrome, COPD, Crohn's disease, irritable bowel syndrome, adult respiratory distress syndrome, osteoporosis, neurodegenerative diseases such as Alzheimer's disease, rheumatoid spondylitis, psoriasis, atherosclerosis, osteoarthritis, multiple myeloma. See for example WO 99/01449, WO 00/63204, WO 01/01986, WO 01/29042, WO 02/046184, WO 02/058695, WO 02/072576, WO 02/072579, WO 03/008413, WO 03/033502, WO 03/087087, WO 03/097062, WO 03/103590, WO 2004/010995, WO 2004/014900, WO 2004/020438, WO 2004/020440, WO 2005/018624, WO 2005/032551, WO 2005/073219 or W02008/107125.

It has now been found that certain 7-phenyl-[1,2,4]triazolo[4,3-a]pyridin-3(2H)-one derivatives are novel potent inhibitors of the p38 mitogen-activated protein kinase and can therefore be used in the treatment or prevention of these diseases.

Further objectives of the present invention are to provide a method for preparing said compounds; pharmaceutical compositions comprising an effective amount of said compounds; said compounds for use as a medicament; the use of the compounds in the manufacture of a medicament for the treatment of pathological conditions or diseases susceptible of being improved by inhibition of the p38 mitogen-activated protein kinase; and methods of treatment of pathological conditions or diseases susceptible to amelioration by inhibition of the p38 mitogen-activated protein kinase comprising the administration of the compounds of the invention to a subject in need of treatment.

Thus, the present invention is directed to new 7-phenyl-[1,2,4]triazolo[4,3-a]pyridin-3(2H)-one derivatives of formula (1) or pharmaceutically acceptable salts or N-oxides thereof 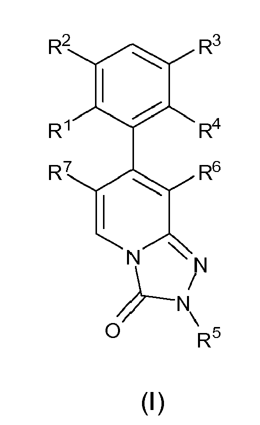 wherein
●R¹ and R² are independently selected from the group consisting of a hydrogen atom, a halogen atom and a C₁-₃ alkyl group;
● either (a) R³ is selected from the group consisting of a -CONR^{a}R^{b} group and a 5 to 7-membered heteroaryl group, wherein said heteroaryl group is optionally substituted with a C₁₋₃alkyl group; and R⁴ is selected from the group consisting of a hydrogen atom, a halogen atom and a C₁₋₃ alkyl group; or (b) R³ together with R⁴ forms a 5 to 7-membered heteroaryl group, wherein said heteroaryl group is optionally substituted with one or more substituents selected from an amino group, a C₁₋₃ alkylamino group and a C₃₋₇ cycloalkylamino;
● R⁵ is selected from the group consisting of a hydrogen atom, a C₁-₆ alkyl group, a C₆-₁₀ aryl group, a 5 to 7-membered heteroaryl group, a C₃₋₇ cycloalkyl group and a 3 to 7-membered heterocyclyl group, wherein said aryl, heteroaryl, cycloalkyl and heterocyclyl groups are optionally substituted with one or more substituents selected from a halogen atom, a C₁-₃ alkyl group, a C₁-₃ alkylsulfonyl group and a C₁₋₄ alkoxycarbonyl group;
● R⁶ and R⁷ are independently selected from the group consisting of a hydrogen atom, a halogen atom and a C₁-₃ alkyl group,
● R^{a} and R^{b} are independently selected from the group consisting of a hydrogen atom, a C₁-₆ alkyl group and a C₃₋₇ cycloalkyl group; or R^{a} and R^{b} together with the nitrogen atom to which they are attached form a 3 to 7-membered heterocyclyl group;
with the proviso that when R¹ is a hydrogen atom, one of R⁶ or R⁷ is other than a hydrogen atom.

As used herein the term C₁-₃ alkyl embraces linear or branched hydrocarbon radicals having 1 to 3 carbon atoms.

Examples of C₁-₃ alkyl groups include methyl, ethyl, n-propyl and i-propyl radicals.

As used herein the term C₁-₆ alkyl embraces linear or branched hydrocarbon radicals having 1 to 6 carbon atoms.

Examples of C₁-₆ alkyl groups include methyl, ethyl, n-propyl, i-propyl, n-butyl, sec-butyl, i-butyl, tert-butyl, n-penthyl, isoamyl and n-hexyl radicals.

As used herein, the term C₃₋₇ cycloalkyl embraces saturated carbocyclic radicals having from 3 to 7 carbon atoms, preferably from 3 to 5 carbon atoms and more preferably from 3 to 4 carbon atoms.

Examples include cyclopropyl, cyclobutyl, cyclopentyl, cyclohexyl and cycloheptyl.

As used herein, the term C₆-C₁₀ aryl group embraces a C₆-C₁₀ monocyclic or polycyclic aryl radical such as phenyl or naphthyl. Phenyl is preferred. A C₆-C₁₀ aryl group is typically unsubstituted or substituted with 1, 2 or 3 substituents. When a C₆-C₁₀ aryl group carries 2 or more substituents, the substituents may be the same or different.

As used herein, the term 5 to 7-membered heteroaryl group embraces a 5 to 7 membered ring system comprising at least one heteroaromatic ring and containing at least one heteroatom, preferably 1, 2 or 3 heteroatoms, selected from O, S and N. A heteroaryl radical may be a single ring or two or more fused rings wherein at least one ring contains a heteroatom.

Examples include pyridyl, pyrazinyl, pyrimidinyl, pyridazinyl, furyl, oxadiazolyl, oxazolyl, imidazolyl, thiazolyl, thiadiazolyl, thienyl, pyrrolyl, triazolyl and pyrazolyl radicals. Pyridyl, oxadiazolyl, oxazolyl, thiadiazolyl and triazolyl radicals are preferred.

A 5 to 7-membered heteroaryl group is typically unsubstituted or substituted with 1, 2 or 3 substituents. When a heteroaryl radical carries 2 or more substituents, the substituents may be the same or different.

As used herein, the term 3 to 7-membered heterocyclyl group embraces a non-aromatic, saturated or unsaturated C₃-C₇ carbocyclic ring, such as a 5, 6 or 7 membered ring, in which one or more, for example 1, 2, or 3 of the carbon atoms, preferably 1 or 2 of the carbon atoms, are each replaced by a heteroatom individually selected from N, O and S. Saturated heterocyclyl radicals are preferred. A heterocyclyl radical may be a single ring or two or more fused rings wherein at least one ring contains a heteroatom.

Examples of heterocyclyl groups include piperidyl, pyrrolidyl, pyrrolinyl, piperazinyl, morpholinyl, thiomorpholinyl, pyrazolinyl, pyrazolidinyl, triazolyl, pyrazolyl, tetrazolyl, imidazolidinyl, imidazolyl, oxiranyl, tetrahydro-2H-pyranyl, 4,5-dihydro-oxazolyl and 3-aza-tetrahydrofuranyl.

As used herein, the term C₁-₄ alkoxycarbonyl group embraces radicals of formula (C₁-₄ alkyl)OC(O)-, wherein said C₁-₄ alkyl is a linear or branched hydrocarbon radical having 1 to 4 carbon atoms.

Examples include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, of which tert-butoxycarbonyl is most preferred.

As used herein, the term halogen atom embraces chlorine, fluorine, bromine or iodine atoms typically a fluorine, chlorine or bromine atom, most preferably chlorine or fluorine. The term halo when used as a prefix has the same meaning.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X-) is associated with the positive charge of the N atom. X- may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and p-toluenesulphonate. X- is preferably an anion selected from chloride, bromide, iodide, sulphate, nitrate, acetate, maleate, oxalate, succinate or trifluoroacetate. More preferably X- is chloride, bromide, trifluoroacetate or methanesulphonate.

As used herein, an N-oxide is formed from the tertiary basic amines or imines present in the molecule, using a convenient oxidising agent.

Typically, R¹ represents a C₁-₃ alkyl group. Preferably R¹ represents a methyl group.

Typically, R² represents a halogen atom, preferably R² represents a fluorine atom.

Typically, R³ represents a -CONR^{a}R^{b} group or R³ together with R⁴ forms a 5 to 7-membered heteroaryl group optionally substituted with an amino group or a C₃-₇ cycloalkylamino group. Preferably R³ represents a -CONR^{a}R^{b} group.

Typically, R^{a} and R^{b} are independently selected from the group consisting of a hydrogen atom, a C₁₋₄ alkyl group and a C₃-₇ cycloalkyl group. Preferably R^{a} and R^{b} are independently selected from a hydrogen atom and a cyclopropyl group.

Typically, R⁴ represents a hydrogen atom or together with R³ form a 5 to 7-membered heteroaryl group optionally substituted with an amino group or a C₃₋₇ cycloalkylamino group. Preferably R⁴ represents a hydrogen atom.

Typically, R⁵ represents a C₁-₆ alkyl group or a phenyl group optionally substituted with one or two halogen atoms.

Typically, R⁶ and R⁷ independently represent a hydrogen atom.

In a preferred embodiment of the present invention, R¹ represents a methyl group; R² represents a fluorine atom; R³ represents a -CONR^{a}R^{b} group; R⁴ represents a hydrogen atom; or R³ together with R⁴ form a 5 to 7-membered heteroaryl group optionally substituted with an amino group or a C₃₋₇, cycloalkylamino group; R⁵ represents a C₁-₆ alkyl group or a phenyl group optionally substituted by one or two halogen atoms; and R⁶ and R⁷ independently represent a hydrogen atom.

In another preferred embodiment of the present invention, R¹ represents a methyl group; R² represents a hydrogen atom or a fluorine atom; R³ represents a 5-methyl-1,3,4-thiadiazol-2-yl group, a 5-methyl-1,3,4-oxadiazol-2-yl group, a 5-methyl-4H-1,2,4-triazol-3-yl group or a cyclopropylcarbamoyl group; R⁴ represents a hydrogen atom or R³ together with R⁴ form an isoxazole ring, wherein said ring is substituted with an amino group or a cyclopropylamino group; R⁵ represents a hydrogen atom, a neopentyl group, a tetrahydro-2H-pyran-4-yl group, a piperidinyl-4-group, a N-(tert-butoxycarbonyl)piperidin-4-yl group, a N-(methylsulfonyl)piperidin-4-yl group, or a phenyl group substituted with a chlorine atom; and R⁶ and R⁷ represent a hydrogen atom.

Particular individual compounds of the invention include:
● *N*-Cyclopropyl-3-fluoro-4-methyl-5-(3-oxo-2,3-dihydro[1,2,4]triazolo[4,3-a]pyridin-7-yl)benzamide;
● *N*-cyclopropyl-3-[2-(2,2-dimethylpropyl)-3-oxo-2,3-dihydro[1,2,4]triazolo[4,3-a]pyridin-7-yl]-5-fluoro-4-methylbenzamide;
● *tert*-Butyl4-[7-f5-[(cyclopropylamino)carbonyl]-3-fluoro-2-methylphenyll-3-oxo-[1,2,4]triazolo[4,3-a]pyridin-2(3H)-yl]piperidine-1-carboxylate;
● *N*-cyclopropyl-3-fluoro-4-methyl-5-(3-oxo-2-piperidin-4-yl-2,3-dihydro[1,2,4]-triazolo[4,3-a]pyridin-7-yl)benzamide;
● *N*-cyclopropyl-3-fluoro-4-methyl-5-f2-[l -(methylsulfonyl)piperidin-4-yl]-3-oxo-2,3-dihydro[1 ,2,4]triazolo[4,3-a]pyridin-7 -yl}benzamide;
● *N*-Cyclopropyl-3-fluoro-4-methyl-5-[3-oxo-2-(tetrahydro-2H-pyran-4-yl)-2,3-dihydro[1,2,4]triazolo[4,3-*a*]pyridin-7-yl]benzamide;
● 3-[2-(2-Chlorophenyl)-3-oxo-2,3-dihydro[1,2,4]triazolo[4,3-a]pyridin-7-yl]-*N-*cyclopropyl-5-fluoro-4-methylbenzamide;
● 2-(2,2-dimethylpropyl)-7-[2-methyl-5-(5-methyl-1,3,4-thiadiazol-2-yl)phenyl][1,2,4]-triazolo[4,3-a]pyridin-3(2H)-one;
● 2-(2,2-dimethylpropyl)-7-[2-methyl-5-(5-methyl-1,3,4-oxadiazol-2-yl)phenyl][1,2,4]-triazolo[4,3-a]pyridin-3(2H)-one;
● 2-(2,2-dimethylpropyl)-7-[3-fluoro-2-methyl-5-(5-methyl-4H-1,2,4-triazol-3-yl)phenyl][1,2,4]triazolo[4,3-a]pyridin-3(2H)-one;
● 7-[3-(cyclopropylamino)-6-methyl-1,2-benzisoxazol-7-yl]-2-(2,2-dimethylpropyl)-[1,2,4]triazolo[4,3-a]pyridin-3(2H)-one; or
● 7-(3-amino-6-methyl-1,2-benzisoxazol-7-yl)-2-(2,2-dimethylpropyl)[1,2,4]triazolo[4,3-a]pyridin-3(2*H*)-one.

The 7-phenyl-[1,2,4]triazolo[4,3-a]pyridin-3(2H)-one derivatives of formula (I) can be converted by known methods into pharmaceutically acceptable salts or N-oxides. Preferred salts are acid addition salts obtainable by treatment with organic or inorganic acids such as fumaric, tartaric, succinic or hydrochloric acid.

The compounds of the present invention may be prepared by methods such as those illustrated in the following Schemes. Solvents, temperatures, pressures and other reaction conditions may readily be selected by one of ordinary skill in the art. Starting materials are commercially available or can be readily prepared by one of ordinary skill in the art using known methods. For all the schemes and compounds described below, R¹, R², R³, R⁴, R⁵, R⁶, R⁷, R^{a} and R^{b} are as described for a compound of general formula (I).

According to a further feature of the present invention, compounds of general formula (I) may be prepared following the synthetic scheme illustrated in Figure 1.

### FIGURE 1

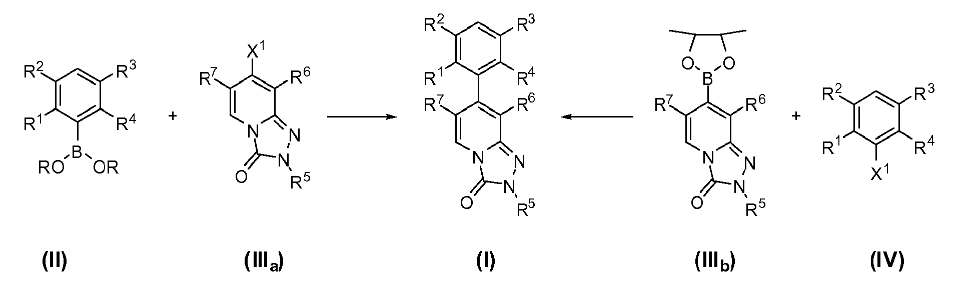

The compounds of formula (I) may be obtained by coupling a haloderivative of formula (IIIₐ) (wherein X₁ is a halogen atom such as iodine, bromine or chlorine) with the corresponding boronic acids or boronates of formula (II) using Suzuki reactions (Miyaura, N.; Suzuki, A. Chem. Rev. 1995, 95, 2457) in the presence of catalysts such as tetrakis(triphenylphosphine)palladium(0) or [1,1'-bis(diphenylphosphino)ferrocene] palladium(II) dichloride: dichloromethane complex (1:1) in solvents such as toluene or dioxane in an aqueous solution of a base such as sodium or cesium carbonate.

Additionally, compounds of formula (I) may be also obtained by coupling a haloderivative of formula (IV) with the corresponding boronate of formula (III_{b}) using the Suzuki conditions described above.

In the particular case where R⁵ is a piperidine, compounds of formula (I) can be prepared by deprotection of the corresponding compounds of formula (I) wherein R⁵ is a piperidine protected with a group such as Boc (tert-butoxycarbonyl) with an acid such as hydrochloridric acid in a solvent such as tetrahydrofuran.

In the particular case where R⁵ is a 1-(methylsulfonyl)piperidine, compounds of formula (I) can be prepared by treating compounds of formula (I) wherein R⁵ is a piperidine with methanesulfonyl chloride and a base such as triethylamine in a solvent such as dichloromethane.

In the particular case where R⁵ is a 2-chlorophenyl, compounds of formula (I) can be prepared by N-Arylation of compounds of formula (I) wherein R⁵ is hydrogen with 1-chloro-2-iodobenzene. These reactions may be catalized by a catalyst such as iodocopper, and a ligand such as *trans-N,N'*-dimethylcyclohexane-1,2-diamine in an organic solvent such as dimethylsulphoxide and in the presence of a base such as potassium carbonate, at a temperature from 90°C to 150°C, using conventional heating or microwave reactor.

Compounds of formula (IV') and (II'), wherein R³ is a -CONR^{a}R^{b} group, may be prepared following the general synthetic procedures disclosed in Figure 2.

### FIGURE 2

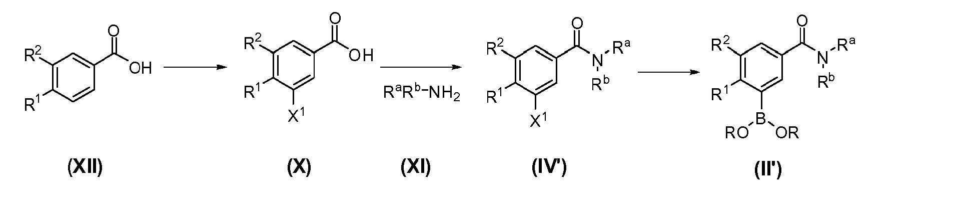

Compounds of general formula (X) are commercially available or can be prepared by direct halogenation of a compound of general formula (XII) with halogenating agents such as N-iodosuccinimide in the presence of a strong acid such as trifluoromethanesulphonic acid.

Compounds of formula (IV') may readily be prepared from a corresponding acid compound of formula (X), by converting the acid to an activated form of the acid, for example the acid chloride, by treatment with, for example, thionyl chloride, and then reacting the activated acid thus formed with an amine compound of formula R^{a}R^{b}-NH₂ (XI) in the presence of a base such as sodium carbonate.

Compounds of formula (II') may be prepared by, for example, reacting a compound of formula (IV'), with bis(pinnacolato)diboron, [1,1'-bis(diphenylphosphino)ferrocene]-dichloropalladium (II) complex (PdCl₂(ppdf)) and potassium acetate in a solvent such as dimethylformamide (DMF), or with isopropylmagnesium chloride and triisopropyl borate in a solvent such as tetrahydrofuran.

In the particular case wherein R³ represents a 5-membered heteroaromatic ring as defined above, intermediates of formula (IV") and (II") may be prepared by following the synthetic routes as depicted in Figure 3.

### FIGURE 3

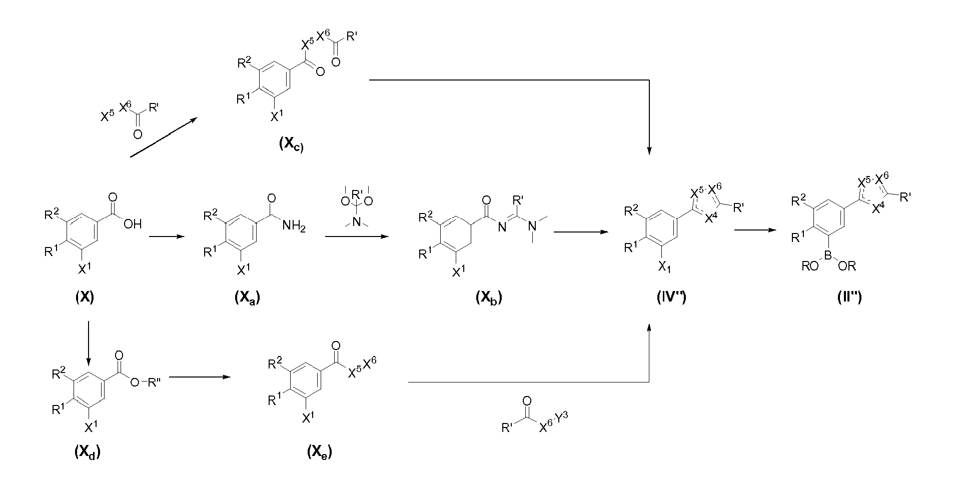

Compounds of formula (Xₐ) may be prepared from compounds of formula (X) by the addition of a suitable activating agent such as 1-(3-dimethylaminopropyl)-3-ethylcarbodiimide hydrochloride, in the presence of 1-hydroxybenzotriazole in a solvent such as N,N'-dimethylformamide at room temperature followed by the addition of a suitable ammonium source such as concentrated aqueous ammonia. Reaction of amides of formula (Xₐ) with a suitable acetal derivative such as N,N'-dimethylformamide dimethylacetal or N,N'-dimethylacetamide dimethylacetal at temperatures ranging from 50 °C to reflux gives the corresponding derivatives of formula (X_{b}). Reaction of compounds of formula (X_{b}) with hydrazine hydrate in a suitable solvent such as ethanol at temperatures ranging from room temperature to reflux gives rise to derivatives of type (IV") wherein X⁴, X⁵ and X⁶ are all nitrogen atoms.

Compounds of formula (X_{c}) may be prepared from compounds of formula (X) by reaction with a corresponding carbohydrazide derivative in a solvent such as methanol at temperatures ranging from room temperature to 75°C. Intermediates of type (X_{c}) may be treated with Lawesson's Reagent in a solvent such as tetrahydrofuran gives derivatives of formula (IV") wherein X⁵ and X⁶ are nitrogen atoms and X⁴ represents a sulfur atom.

Compounds of formula (X) may be transformed into ester derivatives of formula (X_{d}) by, for example, treatment with the corresponding alcohol in the presence of a suitable acid catalyst such as hydrochloric acid at temperatures ranging from room temperature to reflux. Esters of formula (X_{d}) may be treated with hydrazine hydrate in a solvent such as ethanol at temperatures ranging from room temperature to reflux to give hydrazides of formula (Xₑ) which may be transformed into compounds of formula (IV"), wherein X⁵ and X⁶ are nitrogen atoms and X⁴ is an oxygen atom, by treatment with a trialkyl ortho ester such as triethylorthoacetate in a solvent such as acetic acid at temperatures ranging from 70 °C to 150 °C or by addition of the corresponding acid chloride and a base such as triethylamine in an inert solvent such as dichloromethane at a temperature ranging from 0°C to room temperature.

Compounds of formula (II") may be prepared by reacting compounds of formula (IV") following the conditions described for the synthesis of compounds of formula (II') in Figure 2.

In the particular case wherein R³ and R⁴ form a 5-membered heteroaryl group as defined above, intermediates of formula (IV"') may be prepared by following the synthetic routes as depicted in Figure 4.

### FIGURE 4

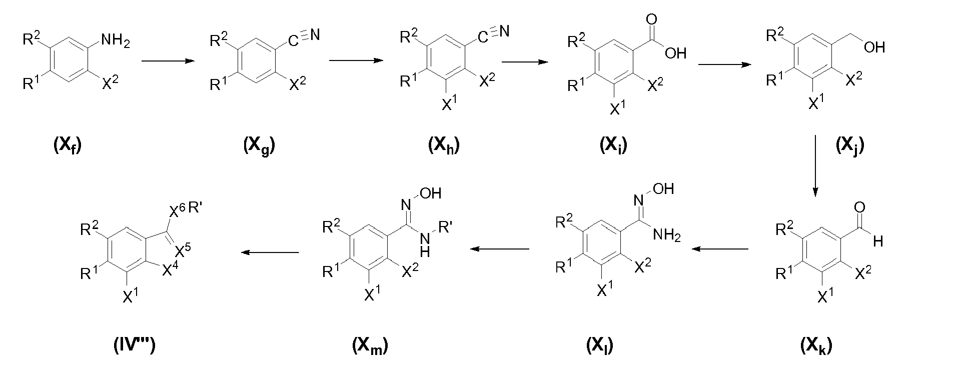

Compounds of formula (X_{g}) can be prepared by Sandmeyer reaction of compounds of formula (X_{f}) (wherein X² is a halogen atom such as fluorine) using Sandmeyer conditions such as addition of sodium nitrite in hydrochloric acid and water followed by addition of cyanocopper and potassium cyanide in water at a temperature from -5 °C to 60°C.

Then, nitrile derivatives of formula (X_{g}) may be halogenated with reactants such as iodide in the presence of butyllithium and 2,2,6,6-tetramethylpiperidine in a solvent such as tetrahydrofuran at temperature from -70 °C to room temperature to give compounds of formula (Xₕ), which may be transformed into compounds of formula (Xᵢ) by treatment with an acid such as sulphuric acid in a solvent such as dioxane at temperatures ranging from 100 °C to 150 °C.

Compounds of formula (Xⱼ) can be prepared by reduction reaction of compounds of formula (Xᵢ) using for instance trimethyl borane and borane dimethyl sulphide in a solvent such as tetrahydrofuran at a temperature from 0 °C to room temperature.

Alcohols of formula (Xⱼ) may be treated with manganese(IV) oxide in a solvent such as dichloromethane at temperatures from room temperature to reflux to give aldehydes of formula (Xₖ) which may be transformed into compounds of formula (X_{I}) by treatment with hydroxylamine hydrochloride in a solvent such as ethanol.

Finally, derivatives of formula (X_{I}) may be treated with N-chlorosuccinimide followed by addition of alkylamine in a solvent such as *N*,*N'*-dimethylformamide at temperature from room temperature to 60 °C to give compounds of formula (Xₘ) which may be transformed into compounds of formula (IV"'), wherein X⁵ and X⁶ are nitrogen atoms and X⁶ is an oxygen atom, by treatment with 2,3,4,6,7,8,9,10-octahydropyrimido[1,2-a]azepine in a solvent such as tetrahydrofuran at temperatures from room temperature to 150 °C, using conventional heating or microwave reactor.

In the particular case where R' is hydrogen, compounds of formula (IV"') can be prepared by reacting compounds of formula (X_{g}) with N-hydroxyacetamide and potassium tert-butoxide in a solvent such as /V,*N'*-dimethylformamide, wherein X⁵ and X⁶ are nitrogen atoms and X⁶ is an oxygen atom.

Compounds of general formula (IIIₐ) and (III_{b}) are prepared following the synthetic scheme illustrated in Figure 5.

### FIGURE 5

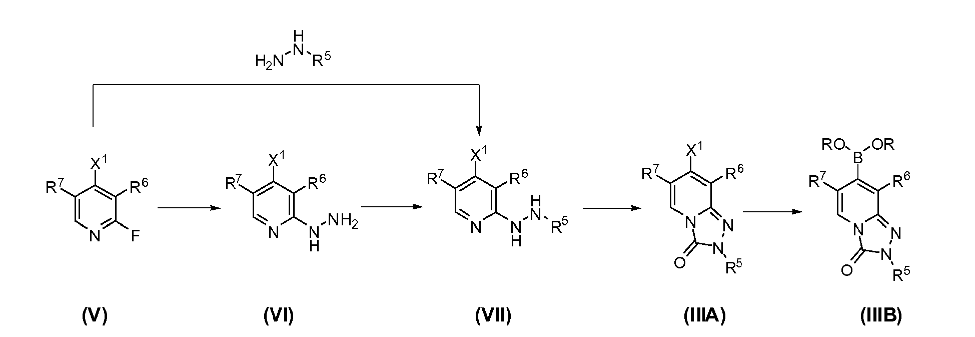

Compounds of formula (VI) can be prepared by reacting compounds of formula (V) with hydrazine in a solvent such as ethanol or pyridine.

Compounds of formula (VII) can be prepared by reductive amination using the corresponding aldehyde in a solvent such as dichloromethane, and followed by addition of a reductive agent such as sodium cyanoborohydride in a solvent such as methanol and acetic acid.

Alternatively, compounds of formula (VII) may be also prepared by reacting compounds of formula (V) with the corresponding hydrazine derivative in a solvent such as ethanol or pyridine.

Compounds of formula (IIIA) can be prepared by reacting compounds of formula (VII) with a carbonylating agent such as triphosgene in an inert solvent such as tetrahydrofuran.

Compounds of formula (IIIB) may be prepared by reacting compounds of formula (IIIA) following the conditions described for the synthesis of compounds of formula (II') in Figure 2.

In the particular case where R⁵ is a hydrogen atom, compounds of formula (IIIA) can be prepared directly from compounds of formula (VI) following the same conditions described for the synthesis of compounds of formula (IIIA) from compounds of formula (VII).

### BIOLOGICAL TESTING

### Inhibition assay

Enzymatic activity assay was performed in 96-well microtiter plates (Corning, catalog number # 3686) using a total volume of 50 µl of an assay buffer composed of 50 mM HEPES (4-(2-hydroxyethyl)-1-piperazineethanesulfonic acid) pH 7.5, 10 mM MgCl₂, 1.75 mM Na₃V0₄.

Various concentrations of the test compound or vehicle controls were pre-incubated for one hour with 0.055 µg/ml of the human p38alfa (SAPKa) enzyme (obtained from University of Dundee). The reaction started by addition of biotinylated ATF2 substrate and ATP in concentrations around their Km values (final concentration 0.62µM and 60µM respectively) and took place for one hour at 25°C. Addition of the detection reagents, streptavidin - XL665 and anti-phosphoresidue antibody coupled to Europium cryptate, caused the juxtaposition of the cryptate and the XL665 fluorophore, resulting in fluorescence energy transfer (FRET).The FRET intensity depends on the amount of bounded cryptate antibody, which is proportional to the extent of substrate phosphorylation. FRET intensity was measured using Victor 2V spectrofluorometer.

Data were analyzed by non-linear regression (Hill equation) to generate a dose-response curve. The calculated IC₅₀ value is the concentration of the test compound that caused a 50% decrease in the maximal FRET intensity.

### Functional assay

The activity of compounds in inhibiting TNFa production was measured in a cellular assay using the human monocytic cel line THP-1. For this purpose, 2x10⁵ cells/well were plated in tissue-culture treated round-bottom 96-well plates in RPMI (containing 10% FCS, L-Gln 2mM, Hepes buffer 10 mM, sodium pyruvate 1 mM, glucose 4.5 g/L, HNaC0₃ 1.5 g/L and beta-mercaptoethanol 50 µM), together with compounds at the desired test concentration and LPS (Sigma, L2630) at a final 10 µg/ml concentration. Compounds were resuspended in 100% DMSO at a concentration of 1 mM and titrated thereof in 10x dilutions in medium. Controls included stimulated cells alone and stimulated cells treated with the highest concentration of compound vehicle (1% DMSO). Cells were incubated for 5h at 37° C in a 5% CO₂ atmosphere. Cell supernatant was recovered by centrifugation and diluted 5-fold prior to testing in a standard human TNFα ELISA (RnD systems).

Data were analyzed by non-linear regression (Hill equation) to generate a dose-response curve. The calculated IC₅₀ value is the concentration of the test compound that caused a 50% decrease in the maximal TNFa production.

Compounds of the present invention are good inhibitors of TNFα production. Preferred 7-phenyl-[1,2,4]triazolo[4,3-a]pyridin-3(2H)-one derivatives derivatives of the invention possess an IC₅₀ value for inhibiting TNFa production of less than 100 µM, preferably less than 10 µM, more preferably less than 1 µM and most preferably less than 100 nM.

Table 1 shows the activities inhibiting p38 assay of some compounds of the present invention.

**TABLE 1**

| **Example** | **p38α IC₅₀ (nM)** |
|---|---|
| 2 | 2 |
| 7 | 1,08 |
| 11 | 12 |

It can be seen from Table 1 that the compounds of formula (I) are potent inhibitors of the p38 mitogen-activated protein kinase. Preferred 7-phenyl-[1,2,4]triazolo[4,3-a]pyridin-3(2H)-one derivatives of the invention possess a IC_{5O} value of inhibition of p38α of less than 1 µM, preferably less than 100 nM, more preferably less than 80 nM and most preferably less than 50 nM.

The 7-phenyl-[1,2,4]triazolo[4,3-a]pyridin-3(2H)-one derivatives of the invention are useful in the treatment or prevention of diseases known to be susceptible to improvement by inhibition of the p38 mitogen-activated protein kinase. Such diseases are, for example rheumatoid arthritis, ischemia-reperfusion injury, cerebral focal ischemia, acute coronary syndrome, asthma, COPD, Crohn's disease, irritable bowel syndrome, adult respiratory distress syndrome, osteoporosis, Alzheimer's disease, rheumatoid spondylitis, psoriasis, atherosclerosis, osteoarthritis or multiple myeloma.

Accordingly, the 7-phenyl-[1,2,4]triazolo[4,3-a]pyridin-3(2H)-one derivatives of the invention and pharmaceutically acceptable salts thereof, and pharmaceutical compositions comprising such compound and/or salts thereof, may be used in a method of treatment of disorders of the human body which comprises administering to a subject requiring such treatment an effective amount of 7-phenyl-[1,2,4]triazolo[4,3-a]pyridin-3(2H)-one derivative of the invention or a pharmaceutically acceptable salt thereof.

When the 7-phenyl-[1,2,4]triazolo[4,3-a]pyridin-3(2H)-one derivatives of the invention are used for the treatment of respiratory diseases such as asthma, chronic obstructive pulmonary disorder, pulmonary fibrosis or emphysema it may be advantageous to use them in combination with other active compounds known to be useful in the treatment of respiratory diseases such as antagonists of M3 muscarinic receptors, β2-agonists, PDE4 inhibitors, corticosteroids, leukotriene D4 antagonists, inhibitors of Egfr-kinase, antagonists of the A_{2B} adenosine receptor, Pl3kδγ inhibitors, NK1 receptor agonists, CRTh2 antagonists, Syk kinase inhibitors, CCR3 antagonists and VLA-4 antagonists.

When the 7-phenyl-[1,2,4]triazolo[4,3-a]pyridin-3(2H)-one derivatives of the invention are used for the treatment of autoimmune diseases such as psoriasis, rheumatoid arthritis, psoriatic arthritis, ankylosing spondilytis, Reiter's syndrome, fibromyalgia, inflammatory bowel disease such as ulcerative colitis and Crohn's disease, multiple sclerosis, diabetes, glomerulonephritis, systemic lupus erythematosus, scleroderma, chronic thyroiditis, Grave's disease, hemolytic anemia, autoimmune gastritis, autoimmune neutropenia, thrombocytopenia, autoimmune chronic active hepatitis, myasthenia gravis, or Addison's disease it may be advantageous to use them in combination with other active compounds known to be useful in the treatment of autoimmune diseases such as PDE4 inhibitors, CysLT1 and/or CysLT2 antagonists, inhibitors of Egfr-kinase, A_{2B} antagonists, Pl3kδγ inhibitors, NK1 receptor agonists, CCR3 antagonists, VLA-4 antagonists and disease modifying antirheumatic drugs (DMARDs).

The present invention also provides pharmaceutical compositions comprising a 7-phenyl-[1,2,4]triazolo[4,3-a]pyridin-3(2H)-one derivative of the invention and another active compound selected from the group consisting of antagonists of M3 muscarinic receptors, β2-agonists, PDE 4 inhibitors, cortiocosteroids, leukotriene D4 antagonists, inhibitors of Egfr-kinase, antagonists of the A_{2B} adenosine receptor, Pl3kγ inhibitors, NK1 receptor agonists, CRTh2 antagonists, Syk kinase inhibitors, CCR3 antagonists, VLA-4 antagonists and disease modifying antirheumatic drugs (DMARDs) such as methotrexate.

Examples of suitable M3 antagonists (anticholinergics) that can be combined with the inhibitors of the p38 mitogen-activated protein kinase of the present invention are tiotropium salts, oxitropium salts, flutropium salts, ipratropium salts, glycopyrronium salts, trospium salts, revatropate, espatropate, 3-[2-Hydroxy-2,2-bis(2-thienyl)acetoxy]-1-(3-phenoxypropyl)-1-azoniabicyclo[2.2.2]octane salts, 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-carboxylic acid endo-8-methyl-8-azabicyclo[3.2.1]oct-3-yl ester salts (DAU-5884), 3-(4-Benzylpiperazin-1-yl)-1-cyclobutyl-1-hydroxy-1-phenylpropan-2-one (NPC-14695), N-[1-(6-Aminopyridin-2-ylmethyl)piperidin-4-yl]-2(R)-[3,3-difluoro-1 (R)-cyclopentyl]-2-hydroxy-2-phenylacetamide (J-104135), 2(R)-Cyclopentyl-2-hydroxy-N-[1-[4(S)-methylhexyl]piperidin-4-yl]-2-phenylacetamide (J-106366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1 (R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-carboxylic acid ester salts, all of them optionally in the form of their racemates, their enantiomers, their diastereomers and mixtures thereof, and optionally in the form of their pharmacologically-compatible acid addition salts. Among the salts chlorides, bromides, iodides and methanesulphonates are preferred.

Examples of suitable β2-agonists that can be combined with the inhibitors of the p38 mitogen-activated protein kinase of the present invention are: arformoterol, bambuterol, bitolterol, broxaterol, carbuterol, clenbuterol, dopexamine, fenoterol, formoterol, hexoprenaline, ibuterol, isoetharine, isoprenaline, levosalbutamol, mabuterol, meluadrine, metaprotenerol, nolomirole, orciprenaline, pirbuterol, procaterol, reproterol, ritodrine, rimoterol, salbutamol, salmefamol, salmeterol, sibenadet, sotenerot, sulfonterol, terbutaline, tiaramide, tulobuterol, GSK-597901, GSK-159797, HOKU-81, (-)-2-[7(S)-[2(R)-Hydroxy-2-(4-hydroxyphenyl)ethylamino]-5,6,7,8-tetrahydro-2-naphthyloxy]-N,N-dimethylacetamide hydrochloride monohydrate, carmoterol, indacaterol and 5-[2-(5,6-diethylindan-2-ylamino)-1-hydroxyethyl]-8-hydroxy-1H-quinolin-2-one, 4-hydroxy-7-[2-{[2-{[3-(2-phenylethoxy)propyl]sulfonyll ethyl]amino}ethyl]-2(3H)-benzothiazoione, 1-(2-fluoro-4-hydroxyphenyl)-2-[4-(1-benzimidazolyl)-2-methyl-2-butylamino]ethanol, 1-[3-(4-methoxybenzylamino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N - dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1 -[2H-5-hydroxy-3-oxo-4H-1 ,4-benzoxazin-8-yl]-2-(4-[3-(4-methoxyphenyl)-1 ,2,4-triazol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluoromethylphenyl)-2-tert-butylamino)ethanol and 1-(4-ethoxycarbonylamino-3-cyano-5-fluorophenyl)-2-(tert-butylamino)ethanol optionally in the form of their racemates, their enantiomers, their diastereomers, and mixtures thereof, and optionally their pharmacologically-compatible acid addition salts and the compounds claimed in Spanish Patent publication numbers ES2265276 and ES2296516. When the β2-agonists are in the form of a salt or derivative It is particularly preferred that it is in a form selected from the sodium salts, sulfobenzoates, phosphates, isonicotinates, acetates, propionates, dihydrogen phosphates, palmitates, pivalates, fumarates, furoates, xinafoates or mixtures thereof.

The following β2-agonists are of special interest for the combination with the compounds of formula (I): arformoterol, bambuterol, bitolterol, broxaterol, carbuterol, clenbuterol, dopexamine, fenoterol, formoterol, hexoprenaline, ibuterol, isoprenaline, levosalbutamol, mabuterol, meluadrine, nolomirole, orciprenaline, pirbuterol, procaterol, (R,R)-formoterol, reproterol, ritodrine, rimoterol, salbutamol, salmeterol, sibenadet, sulfonterol, terbutaline, tulobuterol, GSK-597901, milveterol, carmoterol and indacaterol, optionally in the form of their racemates, their enantiomers, their diastereomers, and mixtures thereof, and optionally their pharmacologically-compatible acid addition salts.

Still most preferred are the following β2-agonists: formoterol, salmeterol, GSK-597901, milveterol and indacaterol, optionally in the form of their racemates, their enantiomers, their diastereomers and mixtures thereof, and optionally their pharmacologically-compatible acid addition salts. Still more preferred are salmeterol and formoterol.

Examples of suitable PDE4 inhibitors that can be combined with the inhibitors of the p38 mitogen-activated protein kinase of the present invention are apremilast, oglemilast, etazolate, tipelukast, tetomilast, roflumilast, RPL-554 (from Verona), ELB-353 (from Biotie Therapie), MEM-1414 (from Memory Pharmaceuticals), DE-103 (from Santen), GPD-1116 (from Aska), GRC-4039 (from Glenmark), BLX-914 (from Orexo), IPL-455903 (from Helicon Therapeutics), GSK-256066 (from GlaxoSmithKline), denbufylline, rolipram, cipamfylline, arofylline, filaminast, piclamilast, lirimilast, (R)-(+)-4-[2-(3-Cyclopentyloxy-4-methoxyphenyl)-2-phenylethyl]pyridine (CP-840 from UCB Celltech), mesopram, drotaverine hydrochloride, cilomilast, N-(3,5-Dichloro-4-pyridinyl)-2-[1-(4-fluorobenzyl)-5-hydroxy-1H-indol-3-yl]-2-oxoacetamide (GW-842470 from GlaxoSmithKline), 9-(2-Fluorobenzyl)-N6-methyl-2-(trifluoromethyl)adenine (NCS-613), 3-[3-(Cyclopentyloxy)-4-methoxybenzyl]-6-(ethylamino)-8-isopropyl-3H-purine hydrochloride (V-11294A), 6-[2-(3,4-Diethoxyphenyl)thiazol-4-yl]pyridine-2-carboxylic acid, 4-[6,7-Diethoxy-2,3-bis(hydroxymethyl)naphthalen-1-yl]-1-(2-methoxyethyl)pyridin-2(1H)-one, 2-carbomethoxy-4-cyano-4-(3-cyclopropylmethoxy-4-difluroromethoxyphenyl)cyclohexan1-one, cis [4-cyano-4-(3-cyclopropylmethoxy-4-difluoromethoxyphenyl)cyclohexan-1-ol, N-[9-Methyl-4-oxo-1-phenyl-3,4,6,7-tetrahydropyrrolo[3,2,1-jk][1,4]benzodiazepin-3(R)-yl]pyridine-4-carboxamide, N-(3,5-Dichloro-4-pyridinyl)-8-methoxyquinoline-5-carboxamide, ONO-6126 (from Ono) and the compounds claimed in the PCT patent application numbers WO03/097613, W02004/058729 A1 and WO 2005/049581 A1.

Examples of suitable corticosteroids and glucocorticoids that can be combined with the inhibitors of the p38 mitogen-activated protein kinase of the present invention are beclomethasone dipropionate, betamethasone 17-valerate, betamethasone butyrate propionate, budesonide, ciclesonide, clobetasol propionate, deflazacort, desonide, dexamethasone, dexamethasone sodium phosphate, difluprednate, fluticasone furoate, fluocinolone acetonide, fluocinonide, fluticasone propionate, hydrocortisone, loteprednol etabonate, methylprednisolone, mometasone furoate, prednisolone, prednisolone sodium metasulfobenzoate, NO-budesonide, prednisone, QAE-397, rimexolone, triamcinolone acetonide, alclometasone dipropionate, betamethasone, betamethasone dipropionate, clocortolone pivalate, deprodone propionate, dexamethasone palmitate, flunisolide, halobetasol propionate, halometasone, halopredone acetate, hydrocortisone aceponate, hydrocortisone acetate, hydrocortisone sodium succinate, hydrocortisone probutate, methylprednisolone aceponate, methylprednisolone suleptanate, naflocort, prednicarbate, prednisolone farnesylate, prednisolone sodium phosphate , tipredane and triamcinolone.

Examples of suitable CysLT1 and/or CysLT2 antagonists that can be combined with the inhibitors of the p38 mitogen-activated protein kinase of the present invention are ibudilast, pranlukast hydrate, tipelukast, montelukast sodium, (E)-8-[2-[4-[4-(4-Fluorophenyl)butoxy]phenyl]vinyl]-2-(1H-tetrazol-5-yl)-4H-benzopyran- 4-one sodium salt (MEN-91507), 2-[N-[4-(4-Chlorophenylsulfonamido)butyl]-N-[3-(4-isopropylthiazol-2-ylmethoxy)benzyl]sulfamoyl]benzoic acid (KP-496), MCC-847 (from AstraZeneca), tomelukast, pobilukast, , zafirlukast, ritolukast, verlukast, sulukast, cinalukast, iralukast sodium, masilukast, 5-[3-[3-(2-Quinolinylmethoxy)phenoxy]propyl]-1H-tetrazole, (3R,4R)-3-[6-(5-Fluorobenzothiazol-2-ylmethoxy)-4-hydroxy-3,4-dihydro-2H-1-benzopyran-3-ylmethyl]benzoic acid, 2-[2-[2-(4-tert-Butylthiazol-2-yl)benzofuran-5-yloxymethyl]phenyl]acetic acid hydrochloride, 5-[2-[4-(Quinolin-2-ylmethoxy)phenoxymethyl]benzyl]-1 H-tetrazole, (E)-2,2-Diethyl-3'-[2-[2-(4-isopropyl)thiazolyl]ethenyl]succinanilic acid; 4-[4-[3-(4-Acetyl-3-hydroxy-2-propylphenoxy)propylsulfonyl]phenyl]-4-oxobutyric acid, [[5-[[3-(4-Acetyl-3-hydroxy-2-propylphenoxy)propyl]thio]-1,3,4-thiadiazol-2-yl]thio]acetic acid, 9-[(4-Acetyl-3-hydroxy-2-n-propylphenoxy)methyl]-3-(1H-tetrazol-5-yl)-4H-pyrido[1,2-a]pyrimidin-4-one, 5-[3-[2-(7-Chloroquinolin-2-yl)vinyl]phenyl]-8-(N,N-dimethylcarbamoyl)-4,6-dithiaoctanoic acid sodium salt; 3-[1-[3-[2-(7-Chloroquinolin-2-yl)vinyl]phenyl]-1-[3-(dimethylamino)-3-oxopropylsulfanyl]methylsulfanyl]propionic acid sodium salt, 6-(2-Cyclohexylethyl)-[1,3,4]thiadiazolo[3,2-a]-1,2,3-triazolo[4,5-d]pyrimidin-9(1H)-one, (R)-3-[2-Methoxy-4-[N-(2-methylphenylsulfonyl)carbamoyl]benzyl]-1-methyl-N-(4,4,4-trifluoro-2-methylbutyl)indole-5-carboxamide, (+)-4(S)-(4-Carboxyphenylthio)-7-[4-(4-phenoxybutoxy)phenyl]-5(Z)-heptenoic acid and the compounds claimed in PCT patent application W02004/043966A1.

Examples of suitable inhibitors of Egfr-kinase that can be combined with the inhibitors of the p38 mitogen-activated protein kinase of the present invention are gefitinib, erlotinib hydrochloride, lapatinib, neratinib, dovitinib lactate, aderbasib, vandetanib, varlitinib, cetuximab, pantimumab, nimotuzumab, zalutumumab, canertinib dihydrochloride, 17-Bromo-20-methoxy-13-methyl-8,9,10,11,12,13,14,19-octahydro-4,6-ethenopyrimido[4,5-b][6,1,12]benzoxadiazacyclopentadecine (JNJ-264833327), 4(R)-Amino-1-[4-(3-methoxyphenylamino)pyrrolo[2,1-f][1,2,4]triazin-5-ylmethyl]piperidin-3(R)-ol (BMS-690514), 7-[4-(3-Ethynylphenylamino)-7-methoxyquinazolin-6-yloxy]heptanohydroxamic acid (CUDC-101), N-[4-(3-Chloro-4-fluorophenylamino)-7-[tetrahydrofuran-3(S)-yloxy]quinazolin-6-yl]-4-(dimethylamino)-2-butenamide (BIBW-2992), N-[4-(3-Chloro-4-fluorophenylamino)-7-[3-methyl-3-(4-methylpiperazin-1-yl)-1-butynyl]quinazolin-6-yl]-2-propenamide (AV-412), EXEL-7647 (from Exilixis), R-7160 (from Roche and Glycart), AZD-4769 (from AstraZeneca), S-222611 (from Shionogi), RO-5083945 (from Roche) and N-[4-(3-Chloro-4-fluorophenylamino)-3-cyano-7-ethoxyquinolin-6-yl]-4-(dimethylamino)-2(E)-butenamide. Examples of suitable antagonists of the A2b adenosine receptor that can be combined with the inhibitors of the p38 mitogen-activated protein kinase of the present invention are CVT-6883 (from CV Therapeutics), 4-(1-butylxanthin-8-yl)benzoic acid (PSB-53), 8-[1-[3-(4-chlorophenyl)-1,2,4-oxadiazol-5-ylmethyl]-1H-pyrazol-4-yl]-1,3-dipropylxanthine, N-(1,3-benzodioxol-5-yl)-2-[5-(1,3-dipropylxanthin-8-yl)-1-methyl-1H-pyrazol-3-yloxy]acetamide (MRE-2029-F20 from King Pharmaceuticals), 8-[4-[5-(2-methoxyphenyl)-1,2,4-oxadiazol-3-ylmethoxy]phenyl]-1,3-dipropylxanthine, 3-[5-(2-methyl-1H-imidazoi-1-yl)-2-(pyrazin-2-ylamino)thiazol-4-yl]benzonitrile, 4-(2,6-dioxo-1-propyl-2,3,6,7-tetrahydro-1H-purin-8-yl)benzenesulfonic acid (PSB-1115), 1-[2-[8-(3-fluorophenyl)-9-methyl-9H-adenin-2-yl]ethynyl]cyclopentanol hydrochloride, N-(2-acetylphenyl)-2-[4-(1,3-dipropylxanthin-8-yl)phenoxy]acetamide (MRS-1668), N-(4-acetylphenyl)-2-[4-(1,3-dipropylxanthin-8-yl)phenoxy]acetamide (MRS-1706), N-(4-cyanophenyl)-2-[4-(1,3-dipropylxanthin-8-yl)phenoxy]acetamide (MRS-1754), 4-(3,4-dichlorophenyl)-5-(4-pyridinyl)thiazol-2-amine (CGH-2466 from Novartis) or the compounds of international patent applications WO 2005/040155 A1, WO2005/100353 A1 and Spanish patent applications ES2274712 and ES2270715.

Examples of suitable antagonists of the Pl3Kδγ inhibitors that can be combined with the inhibitors of the p38 mitogen-activated protein kinase of the present invention are 2-Methyl-2-[4-[3-methyl-2-oxo-8-(3-quinolinyl)-2,3-dihydro-1H-imidazo[4,5-c]quinolin-1-yl]phenyl]propanenitrile (BEZ-235 from Novartis), CAL-101 (from Calistoga Pharmaceuticals) and N-Ethyl-N'-[3-(3,4,5-trimethoxyphenylamino)pyrido[2,3-b]pyrazin-6-yl]thiourea (AEZS-126 from Aeterna Zentaris).

Examples of suitable NK1-receptor antagonists that can be combined with the inhibitors of the p38 mitogen-activated protein kinase of the present invention are vofopitant hydrochloride, aprepitant, casopitant, rolapitant, orvepitant, netupitant, serlopitant, fosaprepitant, nolpitantium besilate, dapitant, lanepitant, ezlopitant, 5-(3,4-Dichlorophenyl)-4(R)-[N-methyl-3,5-bis(trifluoromethyl)benzamido]-N-[2-oxoperhydroazepin-3(R)-yl]-2(E)-pentenamide (DNK-333A from Novartis), 1-[2-[1-[3,5-Bis(trifluoromethyl)benzyl]-5-(4-pyridyl)-1H-1,2,3-triazol-4-yl]pyridin-3-yl]-1-(2-chlorophenyl)methanone (LY-686017 from Lilly), 424887 (from GlaxoSmithKline), TA-5538 (from Mitsubishi Tanabe Pharma), N-[3-(2-Pentylphenyl)propionyl]-threonyl-N-methyl-2,3-dehydrotyrosyl-leucyl-D-phenylalanyl-allo-threonyl-asparaginyl-serine C-1.7-O-3.1 lactone, 1-Methylindol-3-ylcarbonyl-[4(R)-hydroxy]-L-prolyl-[3-(2-naphthyl)]-L-alanine N-benzyl-N-methylamide, (+)-(2S,3S)-3-[2-Methoxy-5-(trifluoromethoxy)benzylamino]-2-phenylpiperidine, (2R,4S)-N-[1-[3,5-Bis(trifluoromethyl)benzoyl]-2-(4-chlorobenzyl)piperidin-4-yl]quinoline-4-carboxamide, [3-[2(R)-[ (R)-[3,5-bis(trifluoromethyl)phenyl]ethoxy]-3(S)-(4-fluorophenyl)-4-morpholinylmethyl]-2,5-dihydro-5-oxo-1H-1,2,4-triazol-1-yl]phosphonic acid 1-deoxy-1-(methylamino)-D-glucitol (1:2) salt, 1'-[2-[2(R)-(3,4-Dichlorophenyl)-4-(3,4,5-trimethoxybenzoyl)morpholin-2-yl]ethyl]spiro[benzo[c]thiophen-1 (3H)-4'-piperidine] 2(S)-oxide hydrochloride and the compound CS-003 described in Eur Respir J 2003, 22(Suppl. 45): Abst P2664.

Examples of suitable CRTh2 antagonists that can be combined with the inhibitors of the p38 mitogen-activated protein kinase of the present invention are AZD-1981 (from AstraZeneca), AZD-8075 (from Astra Zeneca), OC-000459 (from Oxagen), AM-211 (from Amira Pharmaceuticals), AM-461 (from Amira Pharmaceuticals), AZD-5985 (from Astra Zeneca), ACT-129968 (from Actelion), Ramatroban, 2-[5-Fluoro-2-methyl-1-[4-(methylsulfonyl)phenylsulfonyl]-1H-indol-3-yl]acetic acid, [(3R)-4-(4-chlorobenzyl)-7-fluoro-5-(methylsulfonyl)-1,2,3,4tetrahydrocyclopenta[b]indol-3-yl]acetic acid and (1 R,2R,3S,5S)-7-[2-(5-Hydroxybenzothiophen-3-ylcarboxamido)-6,6-dimethylbicyclo[3.1.1 ]hept-3-yl]-5(Z)-heptenoic acid.

Examples of suitable Syk kinase inhibitors that can be combined with the inhibitors of the p38 mitogen-activated protein kinase of the present invention are fosfamatinib (from Rigel), R-348 (from Rigel), R-343 (from Rigel), R-112 (from Rigel), piceatannol, 2-(2-Aminoethylamino)-4-[3-(trifluoromethyl)phenylamino] pyrimidine-5-carboxamide, R-091 (from Rigel), 6-[5-Fluoro-2-(3,4,5-trimethoxyphenylamino)pyrimidin-4-ylamino]-2,2-dimethyl-3,4-dihydro-2H-pyrido[3,2-b][1,4]oxazin-3-one benzenesulfonate (R-406 from Rigel), 1-(2,4,6-Trihydroxyphenyl)-2-(4-methoxyphenyl)ethan-1-one, N-[4-[6-(Cyclobutylamino)-9H-purin-2-ylamino]phenyl]-N-methylacetamide (QAB-205 from Novartis), 2-[7-(3,4-Dimethoxyphenyl)imidazo[1,2-c]pyrimidin-5-ylamino]pyridine-3-carboxamide dihydrochloride (BAY-61-3606 from Bayer) and AVE-0950 (from Sanofi-Aventis).

Examples of CCR3 antagonists that can be combined with the inhibitors of the p38 mitogen-activated protein kinase of the present invention are N-(3-Acetylphenyl)-N'-[(1 R,25)-2-[3(S)-(4-fluorobenzyl)piperid in-1-ylmethyl]cydohexyl]urea benzenesulfonate (DPC-168 from Bristol-Myers Squibb), N-[3-[3(S)-(4-Fluorobenzyl)piperidin-1-yl]-2(S)-hydroxy-1 (R)-methylpropyl]-N'-[3-(1-methyl-1H-tetrazol-5-yl)phenyl]urea (BMS-639623 from Bristol-Myers Squibb), N-[1(R)-[4-(3,4-Dichlorobenzyl)piperidin-1-ylmethyl]-2-methyIpropyl]-N'-(3,4,5-trimethoxyphenyl)urea (RO-1164875 from Roche), N-[1 (S)-[4-(4-Chlorobenzyl)piperidin-1-ylmethyl]-2-hydroxypropyl]-N'-(3,4,5-trimethoxyphenyl)urea (RO-3202947 from Roche), 3-[3-(3-Acetylphenyl)ureido]-2-[4-(4-fluorobenzyl)piperidin-1-ylmethyl]-N-methylbenzamide, 4-(3,4-Dichlorobenzyl)-1-methyl-1-[3-methyl-2(R)-[3-(3,4,5-trimethoxyphenyl)ureido]butyl]piperidinium chloride (RO-1169132), N-[2-[4(R)-(3,4-Dichlorobenzyl)pyrrolidin-2(S)-yl]ethyl]-2-[5-(3,4-dimethoxyphenyl]pyrimidin-2-ylsulfanyl]acetamide (RO-3300802), CRIC-3 (from IPF Pharmaceuticals), 2(R)-[1-[1-(2,4-Dichlorobenzyl)-4(S)-(3-thienyl)pyrrolidin-3(S)-ylmethyl]piperidin-4-ylmethyl]pentanoic acid, 8-[1-(2,4-Dichlorobenzyl)-4(S)-(3-thienyl)pyrrolidin-3(S)-ylmethyl]-3,3-dipropyl-1-oxa-8-azaspiro[4.5]decane-2(S)-carboxylic acid, 11-[1-(2,4-Dichlorobenzyl)-4(S)-(3-thienyl)pyrrolid in-3(S)-ylmethyl]-3,14-dioxa-11-azadispiro[5.1.5.2]pentadecane-15(S)-carboxylic acid, W-56750 (from Mitsubishi Pharma), N-[1 (S)-[3endo-(4-Chlorobenzyl)-8-azabicyclo[3.2.1]oct-8-ylmethyl]-2(S)-hydroxypropyl]-N'-(3,4,5-trimethoxyphenyl)urea, trans-1-(Cycloheptylmethyl)-4-(2,7-dichloro-9H-xanthen-9-ylcarboxamido)-1-methylpiperidinium iodide (UCB-35625 from UCB), GW-782415 (from GlaxoSmithKline), GW-824575 (from GlaxoSmithKline), N-[1'-(3,4-Dichlorobenzyl)-1,4'-bipiperidin-3-ylmethyl]quinoline-6-carboxamide, N-[1-(6-Fluoronaphthalen-2-ylmethyl)pyrrolidin-3(R)-yl]-2-[1-(3-hydroxy-5-methylpyridin-2-ylcarbonyl)piperidin-4-ylidene]acetamide fumarate (YM-355179), 4-[3-[4-(3,4-Dichlorobenzyl)morpholin-2(S)-ylmethyl]ureidomethyl]benzamide, and DIN-106935 (from Bristol-Myers Squibb).

Examples of VLA-4 antagonists that can be combined with the inhibitors of the p38 mitogen-activated protein kinase of the present invention are firategrast, AJM-300 (from Ajinomoto), TBC-4746 (from Encysive Pharmaceuticals and Schering), valategrast, N-[4-[3-(2-Methylphenyl)ureido]phenylacetyl]-L-leucyl-L-aspartyl-L-valyl-L-proline (BIO-1211 from Biogen), 3(S)-[2(S)-[4,4-Dimethyl-3-[4-[3-(2-methylphenyl)ureido]benzyl]-2,5-dioxoimidazolidin-1-yl]-4-methylpentanoylamino]-3-phenylpropionic acid (HMR-1 031 from Sanofi-Aventis), 2(S)-(2,6-Dichlorobenzamido)-3-(2',6'-dimethoxybiphenyl-4-yl)propionic acid (TR-14035 from Mitsubishi Tanabe), RBx-4638 (from Ranbaxy), RBx-7796 (from Ranbaxy), DW-908e (from Daiichi Pharmaceutical), RO-0270608 (from Roche), PS-460644 (from Pharmacopeia) and the compounds claimed in PCT patent application numbers WO 02/057242 A2 and WO 2004/099126 A1.

Examples of disease modifying antirheumatic drugs (DMARDs) that can be combined with the inhibitors of the p38 mitogen-activated protein kinase of the present invention are leflunomide, bucillamine, azathioprine, iguratimod, teriflunomide, (+)-Mefloquine hydrochloride, 4SC-101 (from 4SC), auranofin, cyclosporine, methotrexate, pentostatin, rimacalib hydrochloride, romazarit, salazodine, sulfasalazine, S-2474 (from Shionogi), TA-383 (Mitsubishi Tanabe), SB-273005 (from GlaxoSmithKline), KB-2683 (from Kanebo), CP-690550 (from Pfizer), 3-Deazaadenosine, 6-[5-Fluoro-2-(3,4,5-trimethoxyphenylamino)pyrimidin-4-ylamino]-2,2-dimethyl-3,4-dihydro-2H-pyrido[3,2-b][1,4]oxazin-3-one benzenesulfonate (R-406 from Rigel), AD-827 (from Sosei), BB-2983 (from Vernalis), CPH-82 (from Conpharm), R-1295 (from Roche) and pamapimod.

The combinations of the invention may be used in the treatment of disorders which are susceptible to amelioration by inhibition of the p38 mitogen-activated protein kinase. Thus, the present application encompasses methods of treatment of these disorders, as well as the use of the combinations of the invention in the manufacture of a medicament for the treatment of these disorders, especially for the treatment of rheumatoid arthritis.

The present invention also provides pharmaceutical compositions which comprise, as an active ingredient, at least a 7-phenyl-[1,2,4]triazolo[4,3-a]pyridin-3(2H)-one derivatives of formula (I) or a pharmaceutically acceptable salt thereof in association with a pharmaceutically acceptable excipient such as a carrier or diluent. The active ingredient may comprise 0.001% to 99% by weight, preferably 0.01% to 90% by weight of the composition depending upon the nature of the formulation and whether further dilution is to be made prior to application. Preferably the compositions are made up in a form suitable for oral, topical, inhaled, nasal, rectal, percutaneous or injectable administration. Compositions for oral administration may be in the form of syrups, tablets, capsules, lozenges, controlled-release preparations, fast-dissolving preparations, etc. Compositions for topical administration may be in the form of creams, ointments, lotions, nasal sprays or aerosols, etc). Compositions for administration by injection may be in the form of subcutaneous, intradermic, intramuscular or intravenous compositions. Compositions for administration by inhalation may be in the form of a dry powder, a solution, a dispersion, etc.

The active compounds in the combination, i.e. the inhibitiors of the p38 mitogen-activated protein kinase of the invention, and the other optional active compounds may be administered together in the same pharmaceutical composition or in different compositions intended for separate, simultaneous, concomitant or sequential administration by the same or a different route.

One execution of the present invention consists of a kit of parts comprising an the inhibitiors of the p38 mitogen-activated protein kinase of the present invention together with instructions for simultaneous, concurrent, separate or sequential use in combination with another active compound useful in the treatment of a respiratory disease which responds to inhibition of the p38 mitogen-activated protein kinase.

Another execution of the present invention consists of a package comprising an inhibitior of the p38 mitogen-activated protein kinase of formula (I) and another active compound useful in the treatment of a respiratory disease for the simultaneous, concurrent, separate or sequential use in the treatment of a respiratory disease which responds to the inhibition of the p38 mitogen-activated protein kinase.

The pharmaceutically acceptable excipients which are admixed with the active compound or salts of such compound, to form the compositions of this invention are well-known and the actual excipients used depend *inter alia* on the intended method of administering the compositions.

The diluents which may be used in the preparation of the compositions include those liquid and solid diluents which are compatible with the active ingredient, together with colouring or flavouring agents, if desired. Tablets or capsules may conveniently contain between 1 and 500 mg of active ingredient or the equivalent amount of a salt thereof.

The liquid composition adapted for oral use may be in the form of solutions or suspensions. The solutions may be aqueous solutions of a soluble salt or other derivative of the active compound in association with, for example, sucrose to form a syrup. The suspensions may comprise an insoluble active compound of the invention or a pharmaceutically acceptable salt thereof in association with water, together with a suspending agent or flavouring agent.

Compositions for parenteral injection may be prepared from soluble salts, which may or may not be freeze-dried and which may be dissolved in pyrogen free aqueous media or other appropriate parenteral injection fluid.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred.

Packaging of the formulation may be suitable for unit dose or multi-dose delivery. In the case of multi- dose delivery, the formulation can be pre-metered or metered in use. Dry powder inhalers are thus classified into three groups: (a) single dose, (b) multiple unit dose and (c) multi dose devices.

For single dose inhalers of the first type, single doses have been weighed by the manufacturer into small containers, which are mostly hard gelatin capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients. Other drawbacks related to the use of hard gelatin capsules for inhalation powders are (a) poor protection against moisture uptake from the ambient air; (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported.

Some capsule inhalers have a magazine from which individual capsules can be transferred to a receiving chamber, in which perforation and emptying takes place, as described in WO 92/03175. Other capsule inhalers have revolving magazines with capsule chambers that can be brought in line with the air conduit for dose discharge (e. g. WO91/02558 and GB 2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (e. g. EP0069715) or disks (e. g. GB 2041763; EP 0424790; DE 4239402 and EP 0674533), rotatable cylinders (e. g. EP 0166294; GB 2165159 and WO 92/09322) and rotatable frustums (e. g. WO 92/00771), all having cavities which have to be filled with powder from the container. Other multi dose devices have measuring plungers with a local or circumferential recess to displace a certain volume of powder from the container to a delivery chamber or an air conduit e. g. EP 0505321, WO 92/04068 and WO 92/04928, or measuring slides such as the Novolizer ® SD2FL or Genuair® which is described in the following patent applications: WO 97/000703, WO 03/000325 and WO 03/061742.

Apart from applications through dry powder inhalers the compositions of the invention can also be administered in aerosols which operate via propellant gases or by means of socalled atomisers, via which solutions of pharmacologically-active substances can be sprayed under high pressure so that a mist of inhalable particles results. Such atomisers are described, for example, in W0 91/14468 and WO 97/12687.

Effective doses are normally in the range of 1-2000 mg of active ingredient per day. Daily dosage may be administered in one or more treatments, preferably from 1 to 4 treatments, per day.

The syntheses of the compounds of the invention and of the intermediates for use therein are illustrated by the following Examples (1 to 10) including Preparation Examples (Intermediates 1-13) which do not limit the scope of the invention in any way.

¹H Nuclear Magnetic Resonance Spectra were recorded on a Varian Gemini 300 spectrometer. Melting points were recorded using a Büchi B-540 apparatus. The chromatographic separations were obtained using a Waters 2795 system equipped with a Symmetry C18 (2.1 x 100 mm, 3.5 mm) column. As detectors a Micromass ZMD mass spectrometer using ES ionization and a Waters 996 Diode Array detector were used. The mobile phase was formic acid (0.46 ml), ammonia (0.115 ml) and water (1000 ml) (A) and formic acid (0.4 ml), ammonia (0.1 ml), methanol (500 ml) and acetonitrile (500 ml) (B): initially from 0% to 95% of B in 20 min, and then 4 min. with 95% of B. The reequilibration time between two injections was 5 min. The flow rate was 0.4 ml/min. The injection volume was 5 µl. Diode array chromatograms were processed at 210 nm.

### INTERMEDIATES

### INTERMEDIATE 1

### 7-lodo[1,2,4]triazolo[4,3-a]pyridin-3(2H)-one

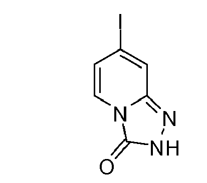

### a) 2-Hydrazinyl-4-iodopyridine

A solution of 2-fluoro-4-iodopyridine (prepared as described in Rocca, P. et al J. Org. Chem. 1993, 58, 7832-7838) (8.72 g, 39.1 mmol) in 80 mL of ethanol was treated with 20 mL of hydrazine hydrate and the mixture was stirred at room temperature (24-25°C) during 20 hours. At the end of the reaction, the mixture was concentrated in vacuum and the resulting solid triturated with a mixture of hexane/diethyl ether affording (8.52 g, 93%) of the desired compound that was used in the following steps without further purification.
LRMS (m/z): 235 (M+1)⁺.
1H NMR (300 MHz, CHLOROFORM-*d*) δ ppm 3.78 (br. s., 2 H), 5.87 (br. s., 1 H), 7.02 (d, J=5.50 Hz, 1 H), 7.19 (s, 1 H), 7.77 (d, J=5.50 Hz, 1 H)

### b) 7-lodo[1,2,4]triazolo[4,3-a]pyridin-3(2H)-one

A solution of bis(trichloromethyl) carbonate (0.71 g, 2.38 mol) in tetrahydrofuran (6 mL) was added dropwise to a stirred solution of 2-hydrazinyl-4-iodopyridine (1.00 g, 4.30 mmol) and triethylamine (2.20 mL, 15.78 mmol) in tetrahydrofuran (6 mL) at 0° C, and the mixture was stirred at room temperature. After overnight stirring, water was added over the reaction, and the mixture was stirred at room temperature. After 1 hour, dichloromethane was added to the mixture and the organic layer was dried (Na₂S0₄) and evaporated to give the desired compound (0.83 g, 58%) that was used in the following steps without further purification.
LRMS (m/z): 260 (M-1)-.
1H NMR (300 MHz, DMSO-*d*₆) δ ppm 6.77 (dd, J=7.30, 1.27 Hz, 1 H) 7.61 (dd, J=7.30, 0.95 Hz, 1 H) 7.78 (dt, J=1.59, 0.79 Hz, 1 H) 12.50 (br. s., 1 H)

### INTERMEDIATE 2

### N-Cyclopropyl-3-fluoro-4-methyl-5-(4,4,5,5-tetramethyl-1,3,2-dioxaborolan-2-yl)benzamide

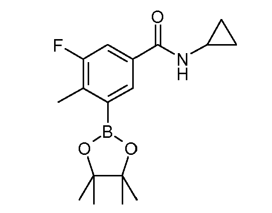

### a) N-Cyclopropyl-3-fluoro-5-iodo-4-methylbenzamide

*N*-lodosuccinimide (3.31 g, 14.71 mmol) was added in portions to a solution of 3-fluoro-4-methylbenzoic acid (2.27 g, 14.77 mmol) in trifluoromethanesulphonic acid (15 mL) at 0° C over 3 hours and the mixture was allowed to warm to room temperature overnight. The reaction mixture was poured into ice/water and the precipitate was collected by filtration and washed with water. The solid was dissolved in ethyl acetate, washed with aqueous sodium thiosulphate (x2) and brine, dried (Na₂S0₄) and the solvent was removed under vacuum. The residue obtained was treated with thionyl chloride (20 mL) and heated at 100° C for 2.5 hours. Excess thionyl chloride was removed under vacuum and the residue was dissolved in dichloromethane (20 mL). Sodium carbonate (3.7 g, 34.90 mmol) and cyclopropylamine (1.9 mL, 27.42 mmol) were added to the solution and the mixture was stirred at room temperature for 72 hours. The mixture was filtered and the residue was washed with dichloromethane and ethyl acetate. The combined filtrate and washings were concentrated under vacuum. The residue was purified by flash chromatography (10:1 hexanes/ethyl acetate to 5:1 hexanes/ethyl acetate) to give the title compound (2.13 g, 45%).
LRMS (m/z): 320 (M+1)⁺.

### b) N-Cyclopropyl-3-fluoro-4-methyl-5-(4,4,5,5-tetramethyl-1,3,2-dioxaborolan-2-yl)benzamide

*N*-Cyclopropyl-3-fluoro-5-iodo-4-methylbenzamide (Intermediate 2a, 1.00 g, 3.13 mmol), 4,4,5,5,4',4',5',5'-octamethyl-[2,2']bi[[1,3,2]dioxaborolanyl] (1.2 g, 4.42 mmol), potassium acetate (1.23 g, 12.55 mmol) and [1,1'-bis(diphenylphosphino)ferrocene]dichloro-palladium(II) complex with dichloromethane (1:1) (0.15 g, 0.19 mmol) were mixed in DMF (38 mL) and heated at 110°C for 18 hours. The cooled reaction was concentrated in vacuo and the residue was purified by flash chromatography (6:1 hexanes/ethyl acetate) to give the title compound (0.71 g, 57%).
LRMS (m/z): 320 (M+1)⁺.

### INTERMEDIATE 3

### 2-(2,2-Dimethylpropyl)-7-iodo[1,2,4]triazolo[4,3-a]pyridin-3(2H)-one

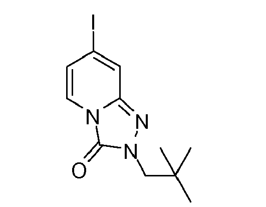

### a) 2-[2-(2,2-Dimethylpropyl)hydrazino]-4-iodopyridine

Pivalaldehyde (0.56 mL, 5.01 mmol) was added to a solution of 2-hydrazinyl-4-iodopyridine (Intermediate 1a, 1.00 g, 4.14 mmol) in dichloroethane (40 mL) and the mixture was stirred at room temperature. After 1 hour, the solvent was removed under vacuum. Sodium cyanoborohydride (0.41 g, 6.31 mmol) was added to a solution of the residue obtained in methanol (42 mL) and acetic acid (7 mL) and the mixture was stirred at room temperature. After 1 hour, the solvent was removed under vacuum. The crude was purified by flash chromatography (9:1 hexanes/propan-2-ol) to give the title compound (1.04 g, 81%).
LRMS (m/z): 306 (M+1)⁺.
1H NMR (300 MHz, CHLOROFORM-*d*) δ ppm 0.99 (s, 9 H) 2.62 (s, 2 H) 3.84 (br. s., 1 H) 5.87 (br. s., 1 H) 6.94 - 7.08 (m, 2 H) 7.70 (d, 1 H)

### b) 2-(2,2-Dimethylpropyl)-7-iodo[1,2,4]triazolo[4,3-a]pyridin-3(2H)-one

Obtained (98%) from 2-[2-(2,2-dimethylpropyl)hydrazino]-4-iodopyridine (Intermediate 3a) following the experimental procedure as described in Intermediate 1 b.
LRMS (m/z): 332 (M+1)⁺.

### INTERMEDIATE 4

### tert-Butyl 4-(7-iodo-3-oxo[1,2,4]triazolo[4,3-a]pyridin-2(3H)-yl)piperidine-1-carboxylate

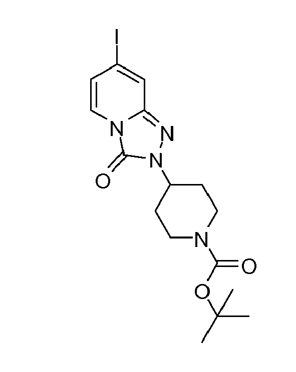

### a) tert-Butyl 4-[2-(4-iodopyridin-2-yl)hydrazino]piperidine-1-carboxylate

Obtained (99%) from 2-hydrazinyl-4-iodopyridine (Intermediate 1a) and *tert-*butyl 4-oxopiperidine-1-carboxylate following the experimental procedure as described in Intermediate 3a.
LRMS (m/z): 419 (M+1)⁺.

### b) tert-Butyl 4-(7-iodo-3-oxo[1,2,4]triazo)o[4,3-a]pyridin-2(3H)-y))piperidine-1 - carboxylate

Obtained (69%) from *tert-*butyl 4-[2-(4-iodopyridin-2-yl)hydrazino]piperidine-l-carboxylate (Intermediate 4a) following the experimental procedure as described in Intermediate 1 b.
LRMS (m/z): 445 (M+1)⁺.

### INTERMEDIATE 5

### 7-lodo-2-(tetrahydro-2H-pyran-4-yl)[1,2,4]triazolo[4,3-a]pyridin-3(2H)-one

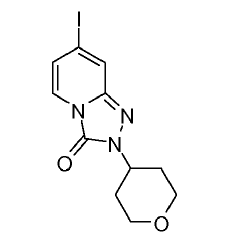

### a) 4-iodo-2-[2-(tetrahydro-2H-pyran-4-yl)hydrazino]pyridine

Obtained (99%) from 2-hydrazinyl-4-iodopyridine (Intermediate 1a) and tetrahydro-4*H-*pyran-4-one following the experimental procedure as described in Intermediate 3a.
LRMS (m/z): 320 (M+1)⁺.

### b) 7-lodo-2-(tetrahydro-2H-pyran-4-yl)[1,2,4]triazolo[4,3-a]pyridin-3(2H)-one

Obtained (93%) from 4-iodo-2-[2-(tetrahydro-2H-pyran-4-yl)hydrazino]pyridine (Intermediate 5a) following the experimental procedure as described in Intermediate 1 b.
LRMS (m/z): 346 (M+1)⁺.
1H NMR (300 MHz, CHLOROFORM-*d*) δ ppm 1.85 (dd, *J*=12.50, 1.51 Hz, 2 H) 2.19 (dd, J=12.22, 3.98 Hz, 2 H) 3.55 (td, *J*=11.95, 1.65 Hz, 2 H) 4.10 (d, *J*=11.81 Hz, 2 H) 4.42 - 4.58 (m, 1 H) 6.71 (dd, J=7.28, 1.51 Hz, 1 H) 7.51 (dd, J=7.42, 0.82 Hz, 1 H) 7.59 (d, 1 H)

### INTERMEDIATE 6

### 2-(3-lodo-4-methylphenyl)-5-methyl-1,3,4-thiadiazole

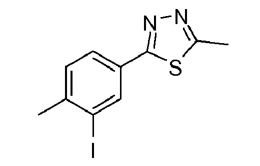

### a) N'-Acetyl-3-iodo-4-methylbenzohydrazide

A solution of 3-iodo-4-methylbenzoic acid (1.01 g, 3.73 mmol), N-(3-dimethylaminopropyl)-N'-ethylcarbodiimide hydrochloride (0.72 g, 3.74 mmol) and 1H-benzo[d][1,2,3]triazol-1-ol hydrate (0.57 g, 3.76 mmol) in N,N'-dimethylformamide (4 mL) were sequentially added to a stirred solution of acetohydrazide (0.29 g, 3.89 mmol) and triethylamine (0.55 mL, 3.95 mmol) in N,N'-dimethylformamide (4 mL) at room temperature. After stirring for 2 hours, the mixture was concentrated in vacuo and water was added to the mixture. The suspension was filtered and the filter cake was washed with additional water. The filtrate (0.83 g, 63%) was used in the following steps without further purification.
LRMS (m/z): 319 (M+1)⁺.

### b) 2-(3-lodo-4-methylphenyl)-5-methyl-1,3,4-thiadiazole

Lawesson's Reagent (0.75 g, 1.85 mmol) was added to a solution of *N*'-acetyl-3-iodo-4-methylbenzohydrazide (Intermediate 6a, 0.55 g, 1.54 mmol) in tetrahydrofuran (8 mL) and the mixture was stirred at 60° C. After 1 hour, a solution of sodium hydroxide (0.60 g) in water (40 mL) was added to the reaction mixture at 0° C and the precipitate that formed was collected by filtration. The filtrate (0.37 g, 74%) was used in the following steps without further purification.
LRMS (m/z): 317 (M+1)⁺.
1H NMR (300 MHz, CHLOROFORM-*d*) δ ppm 2.50 (s, 3 H) 2.83 (s, 3 H) 7.34 (d, J=7.69 Hz, 1 H) 7.82 (dd, J=7.83, 1.79 Hz, 1 H) 8.40 (d, J=1.92 Hz, 1 H)

### INTERMEDIATE 7

### 2-(2,2-dimethylpropyl)-7-(4,4,5,5-tetramethyl-1,3,2-dioxaborolan-2-yl)[1,2,4]triazolo[4,3-a]pyridin-3(2H)-one

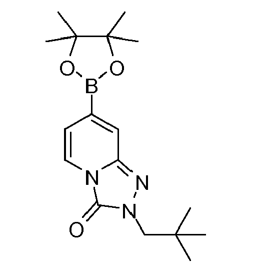

Obtained from 2-(2,2-dimethylpropyl)-7-iodo[1,2,4]triazolo[4,3-a]pyridin-3(2*H*)-one (Intermediate 3b) following the experimental procedure as described in Intermediate 2b.
LRMS (m/z): 332 (M+1)⁺.

### INTERMEDIATE 8

### [2-methyl-5-(5-methyl-1,3,4-oxadiazol-2-yl)phenyl]boronic acid

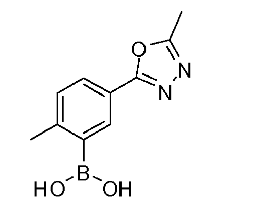

### a) Methyl 3-iodo-4-methylbenzoate

A suspension of 3-iodo-4-methylbenzoic acid (15.00 g, 60.00 mmol) and a 4M solution of hydrochloric acid in dioxane (20.00 mL, 80.0 mmol) in methanol (20 mL) was heated in a sealed tube at 80 °C with stirring. After stirring for 3 days, the mixture was cooled and ethyl acetate and saturated aqueous potassium carbonate solution were added. The organic layer was washed with brine, dried (MgSO₄) and evaporated in vacuo to give the title compound (15.00 g, 95%).
LRMS (m/z): 277 (M+1)⁺.

### b) 3-lodo-4-methylbenzohydrazide

Hydrazine monohydrate (27.00 mL, 55.00 mmol) was added to a stirred solution of methyl 3-iodo-4-methylbenzoate (Intermediate 8a, 15.00 g, 50.00 mmol) in ethanol (250 mL) and the mixture was stirred at 80 °C. After overnight stirring, the mixture was cooled, concentrated in vacuo and the precipitate that formed was collected by filtration. The filtrate (12.89 g, 86%) was used in the following steps without further purification.
LRMS (m/z): 277 (M+1)⁺.
1H NMR (300 MHz, DMSO-*d*₆) δpm 2.38 (s, 3 H), 4.47 (br. s., 2 H), 7.38 (d, J = 8.00 Hz, 1 H), 7.74 (dd, J = 8.00 and 2.00 Hz, 1 H), 8.23 (d, J = 2.00 Hz, 1 H), 9.79 (br. s., 1 H)

### c) 2-(3-lodo-4-methylphenyl)-5-methyl-1,3,4-oxadiazole

A stirred mixture of 3-iodo-4-methylbenzohydrazide (Intermediate 8b, 5.00 g, 18.10 mmol) and triethyl orthoacetate (11.00 mL, 58.20 mmol) in acetic acid (30 mL) were heated to 150 °C in a sealed tube. After 3 hours, the mixture was cooled and concentrated in vacuo. Aqueous sodium hydrogen carbonate solution was added and the mixture was extracted with dichloromethane. The organic solution was dried (MgS0₄) and the solvent was removed in vacuo. The crude material was purified by flash chromatography (2:1 hexanes/ethyl acetate) to give the title compound (4.26 g, 78%) as a white solid.
LRMS (m/z): 301 (M+1)⁺.
1H NMR (300 MHz, CHLOROFORM-*d*) δ ppm 2.50 (s, 3 H) 2.62 (s, 3 H) 7.35 (d, J=7.97 Hz, 1 H) 7.91 (d, J=7.97 Hz, 1 H) 8.46 (s, 1 H)

### d) [2-Methyl-5-(5-methyl-1 ,3,4-oxadiazol-2-yl)phenyl]boronic acid

Isopropylmagnesium chloride (2.0 M in tetrahydrofuran, 10 mL, 20.00 mmol) was added dropwise over 20 minutes to a stirred solution of 2-(3-lodo-4-methylphenyl)-5-methyl-1,3,4-oxadiazole (Intermediate 8c, 4.0 g, 13.30 mmol) in tetrahydrofuran (40 mL) at -10 °C. The mixture was stirred for 1 hour at - 10 °C and then triisopropyl borate (4.71 mL, 20.0 mmol) was added dropwise over 10 minutes and the mixture was stirred for 1 hour at - 10 °C. Then, the mixture was warmed to room temperature and stirred overnight. Water was then added, the mixture was cooled by means of an ice-water bath and the pH was adjusted to 3-4 with 2M aqueous hydrochloric acid. The mixture was extracted with ethyl acetate and the organic layer was washed with brine, dried (MgSO₄) and evaporated. The residue was triturated with diethyl ether and the solid was filtered and dried to give the title compound (1.92 g, 66%) as a white solid.
LRMS (m/z): 219 (M+1)⁺.
1H NMR (300 MHz, DMSO-*d*₆) δ ppm 2.46 (s, 3 H), 2.56 (s, 3 H), 7.32 (d, J = 8.00 Hz, 1 H), 7.81 (dd, J = 8.00 and 2.00 Hz, 1 H), 8.02 (d, J = 2.00 Hz, 1 H), 8.25 (br. s., 2 H)

### INTERMEDIATE 9

### 3-(3-Fluoro-5-iodo-4-methylphenyl)-5-methyl-4H-1,2,4-triazole

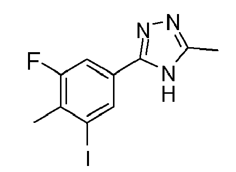

### a) 3-Fluoro-5-iodo-4-methylbenzoic acid

*N*-lodosuccinimide (3.31 g, 14.70 mmol) was added in portions over a 3-hour period to a stirred solution of 3-fluoro-4-methylbenzoic acid (2.27 g, 14.70 mmol) in trifluoromethanesulphonic acid (15 mL) at 0 °C. The mixture was warmed to room temperature, stirred overnight and then poured into an ice-water mixture and the precipitate that formed was collected by filtration and washed with water. The solid was dissolved in ethyl acetate, washed with aqueous sodium thiosulphate and brine, dried (MgSO₄ and dried to give the title compound (3.45 g, 59%) as a solid.
LRMS (m/z): 279 (M-1)⁻.
1H NMR (300 MHz, CHLOROFORM-*d*) δ ppm 2.41 (s, 3 H), 2.86 (m, 1 H), 6.30 (br. s , 1 H), 7.72 (m, 1 H), 8.34 (m, 1 H)

### b) 3-Fluoro-5-iodo-4-methylbenzamide

N-(3-dimethylaminopropyl)-N'-ethylcarbodiimide hydrochloride (2.01 g, 10.49 mmol), 1H-benzo[d][1,2,3]triazol-1-ol hydrate (1.59 g, 10.52 mmol) and 32% aqueous ammonium hydroxide solution (0.55 mL, 13.97 mmol) were sequentially added to a stirred solution of 3-fluoro-5-iodo-4-methylbenzoic acid (Intermediate 9a, 1.96 g, 7.00 mmol) in N,N'-dimethylformamide (11 mL) at room temperature. After stirring overnight, the mixture was concentrated in vacuo and then water was added to the crude. The solid that formed was filtered, washed with water, 1 M aqueous sodium hydroxide solution, 1 M aqueous hydrogen chloride solution and water, and dried in vacuo to give the title compound (1.74 g, 89%) as a white solid.
LRMS (m/z): 280 (M+1)⁺.
1H NMR (300 MHz, DMSO-*d*₆) δ ppm 2.34 (s, 3 H) 7.56 (br. s., 1 H) 7.67 (d, J=10.44 Hz, 1 H) 8.09 (br. s., 1 H) 8.18 (s, 1 H)

### c) N-[(1 E)-1 -(Dimethy)amino)ethy)idene]-3-f)uoro-5-iodo-4-methy)benzamide

A mixture of 3-fluoro-5-iodo-4-methylbenzamide (Intermediate 9b) and (1,1-dimethoxyethyl)dimethylamine was heated at 120 °C in a sealed tube. After 2 hours, the reaction mixture was cooled and the solvent was evaporated under reduced pressure to give the title compound (1.84 g, 100%) as an oil.
LRMS (m/z): 349 (M+1)⁺.
1H NMR (300 MHz, CHLOROFORM-*d*) δ ppm 2.33 (s, 3 H) 2.39 (d, J=2.47 Hz, 3 H) 3.15 (s, 3 H) 3.21 (s, 3 H) 7.75 (dd, J=10.16, 1.10 Hz, 1 H) 8.36 (s, 1 H)

### d) 3-(3-Fluoro-5-iodo-4-methylphenyl)-5-methyl-4H-1,2,4-triazole

Hydrazine monohydrate (0.45 mL, 9.23 mmol) was added to a stirred solution of *N*-[(1*E*)-1-(dimethylamino)ethylidene]-3-fluoro-5-iodo-4-methylbenzamide (Intermediate 9c, 2.17 g, 6.23 mmol) in acetic acid (6 mL) and the mixture was stirred and heated to 90 °C. After 2 hours, the mixture was concentrated in vacuo and water was added to the residue. The solid that formed was filtered, washed with water and hexanes, and dried in vacuo to give the title compound (1.57 g, 91%) as a solid.
LRMS (m/z): 318 (M+1)⁺.
1H NMR (300 MHz, DMSO-d*6*) δ ppm 2.33 (s, 3 H) 2.39 (s, 3 H) 7.67 (d, J=10.44 Hz, 1 H) 8.25 (s, 1 H)

### INTERMEDIATE 10

### N-Cyclopropyl-7-iodo-6-methyl-1,2-benzisoxazol-3-amine

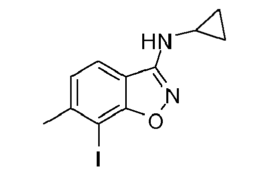

### a) 2-Fluoro-4-methylbenzonitrile

A solution of sodium nitrite (4.22 g, 61.16 mmol) in water (40 mL) was added slowly to a stirred suspension of (2-fluoro-4-methylphenyl)amine (4.60 mL, 40.73 mmol) in hydrogen chloride (1M, 340 mL) at -5 °C, and the mixture was stirred at this temperature. After 10 minutes, sodium bicarbonate was added until the pH was 7-8. The reaction mixture was added slowly to a stirred solution of cyanocopper (12.22 g, 136.44 mmol) and potassium cyanide (8.10 g, 124.39 mmol) in water (150 mL). The reaction was warmed to 60 °C. After heating for 2 hours, ethyl acetate was added to the mixture and the aqueous layer was extracted with more ethyl acetate. The organic layer was washed with brine, dried (Na₂S0₄) and evaporated. Purification of the crude by flash chromatography (85:15 hexanes/ethyl acetate) gave the title compound (2.56 g, 46%).
LRMS (m/z): 136 (M+1)⁺.
1H NMR (300 MHz, CHLOROFORM-*d*) δ ppm 2.45 (s, 3 H) 6.98 - 7.12 (m, 2 H) 7.51 (t, J=6.87 Hz, 1 H)

### b) 2-Fluoro-3-iodo-4-methylbenzonitrile

Butyllithium (2.5M in Hexanes, 10.20 mL, 25.43 mmol) was added slowly to a stirred solution of 2,2,6,6-tetramethylpiperidine (4.72 mL, 27.97 mmol) in tetrahydrofuran (30 mL) at -70 °C, and the mixture was stirred at -50 °C. After 50 minutes, 2-fluoro-4-methylbenzonitrile (Intermediate 10a, 2.76 g, 16.95 mmol) was added to the reation mixture at -70 °C and the reaction was stirred at -50 °C. After 50 minutes, iodide (6.02 g, 23.73 mmol) in tetrahydrofuran (10 mL) was added to the reation mixture at -70 °C and slowly the reation was warmed to room temperature. After 3 hours at room temperature, aqueous sodium hydrogensulfate solution (150 mL) was added to the reaction and the mixture was extracted with ethyl acetate. The organic layer was washed with brine, dried (Na₂S0₄) and evaporated. Purification of the crude by flash chromatography (95:5 hexanes/ethyl acetate to 8:2 hexanes/ethyl acetate) gave the title compound (3.54 g, 80%).
LRMS (m/z): 279 (M+18t
1H NMR (300 MHz, CHLOROFORM-*d*) δ ppm 2.56 (s, 3 H) 7.15 (d, J=7.97 Hz, 1 H) 7.49 (dd, J=7.83, 6.45 Hz, 1 H)

### c) 2-Fluoro-3-iodo-4-methylbenzoic acid

Sulfuric acid (60%, 80.00 mL, 0.49 mol) was slowly added to a stirred solution of 2-fluoro-3-iodo-4-methylbenzonitrile (Intermediate 10b, 15.80 g, 0.06 mol) in dioxane (50 mL) and the mixture was stirred at 115 °C. After 48 hours, the mixture was cooled and poured onto ice-water. The aqueous layer was extracted with ethyl acetate, and the organic layer was washed with water, brine, dried (Na₂SO₄) and evaporated to give the title compound (15.66 g, 92%) as an off-white solid.
LRMS (m/z): 279 (M-1)⁻.
1H NMR (300 MHz, DMSO-*d*₆) δ ppm 2.48 (s, 3 H) 7.28 (d, J=8.24 Hz, 1 H) 7.75 (t, J=7.83 Hz, 1 H)

### d) (2-Fluoro-3-iodo-4-methylphenyl)methanol

Trimethyl borate (6.5 mL, 0.06 mol) was added to a stirred solution of 2-fluoro-3-iodo-4-methylbenzoic acid (Intermediate 10c, 15.78 g, 0.06 mol) in tetrahydrofuran (80 mL) under an argon atmosphere and the mixture was stirred at room temperature. After 15 minutes, a solution of borane dimethyl sulphide (11.50 mL, 0.12 mol) in tetrahydrofuran (10 mL) was added to the reaction mixture at 0 °C. The reaction was stirred at room temperature for 3 hours. After that, methanol (5 mL) was added slowly to the mixture. After 30 minutes, the solvent was evaporated, and ethyl acetate was added to the residue, the organic layer was washed with saturated aqueous sodium hydrogen carbonate solution and water, dried (Na₂SO₄) and evaporated to give the title compound (14.60 g, 97%) as a white solid.
1H NMR (300 MHz, CHLOROFORM-*d*) δ ppm 2.47 (s, 3 H) 4.74 (s, 2 H) 7.05 (d, J=7.97 Hz, 1 H) 7.28 (t, J=7.55 Hz, 1 H)

### e) 2-Fluoro-3-iodo-4-methylbenzaldehyde

Manganese(IV) oxide (2.55 g, 24.93 mmol) was added in portions to a stirred solution of (2-fluoro-3-iodo-4-methylphenyl)methanol (Intermediate 10d, 0.77 g, 2.90 mmol) in dichloromethane (25 mL) at room temperature. After stirring overnight at 45°C, the mixture was cooled and filtered. The filtrate was concentrated in vacuo to give the title compound (0.60 g, 79%) as a yellow solid.
1H NMR (300 MHz, CHLOROFORM-*d*) δ ppm 2.57 (s, 3 H) 7.20 (d, J=7.69 Hz, 1 H) 7.75 (t, J=7.42 Hz, 1 H) 10.30 (s, 1 H)

### f) 2-Fluoro-3-iodo-4-methylbenzaldehyde oxime

To a suspension of the 2-fluoro-3-iodo-4-methylbenzaldehyde (Intermediate 10e, 1.47 g, 5.57 mmol) in ethanol (12 mL), a warm aqueous hydroxylamine hydrochloride solution (50%, 5.90 mL, 45.45 mmol) was added. The mixture was warmed with a heat gun until all solids were dissolved and was stirred at room temperature. After 2 hours, the mixture was heated until all the solids were dissolved and then cooled to room temperature. The mixture was poured into water and the solid was filtered, washed with water and dried in vacuo to give the title compound (1.48 g, 96%) as an off-white solid.
LRMS (m/z): 280 (M+1)⁺.
1H NMR (300 MHz, CHLOROFORM-*d*) δ ppm 2.49 (s, 3 H) 7.06 (d, J=7.97 Hz, 1 H) 7.62 (t, J=7.55 Hz, 1 H) 7.74 (br. s., 1 H) 8.34 (s, 1 H)

### g) N-Cyclopropyl-2-fluoro-N'-hydroxy-3-iodo-4-methylbenzenecarboximidamide

N-chlorosuccinimide (0.38 g, 2.79 mmol) was added to a stirred solution of 2-fluoro-3-iodo-4-methylbenzaldehyde oxime (Intermediate 10f, 1.48 g, 5.30 mmol) in *N,N'-*dimethylformamide (3 mL) and the mixture was stirred at 55 °C for 5 minutes. Further N-chlorosuccinimide (0.37 g, 2.72 mmol) was added and the mixture was stirred at 55 °C for 10 minutes more. After cooling to room temperature, the solvent was evaporated under reduced pressure. The residue obtained was dissolved in tetrahydrofuran and a solution of cyclopropylamine (1.88 mL, 26.59 mmol) in tetrahydrofuran was added, and the mixture reaction was stirred at room temperature overnight. The solvent was then evaporated under reduced pressure, and the residue was partitioned between water and ethyl acetate. The organic layer was washed with brine, dried (MgSO₄ and concentrated to yield the title compound (1.68 g, 95%) as a beige solid.
LRMS (m/z): 335 (M+1)⁺.

### h) N-Cyclopropyl-7-iodo-6-methyl-1,2-benzisoxazol-3-amine

2,3,4,6,7,8,9,10-Octahydropyrimido[1,2-a]azepine (0.90 mL, 6.02 mmol) was added to a stirred solution of *N*-cyclopropyl-2-fluoro-*N'*-hydroxy-3-iodo-4-methylbenzenecarboximidamide (Intermediate 10g, 1.68 g, 5.04 mmol) in tetrahydrofuran (8.5 mL), the mixture was heated at 150 °C for 5.5 hours in a Biotage Initiator Microwave Synthesizer. The mixture was then cooled and the solvent was evaporated. Purification of the residue by flash chromatography (3:1 hexanes/ethyl acetate) gave the title compound (0.90 g, 57%) as a beige solid.
LRMS (m/z): 315 (M+1)⁺.
1H NMR (300 MHz, CHLOROFORM-*d*) δ ppm 0.71 (dd, J=3.57, 2.47 Hz, 2 H) 0.82 - 0.92 (m, 2 H) 2.58 (s, 3 H) 2.77 - 2.90 (m, J=6.90, 2.99, 2.88, 2.75, 1.24 Hz, 1 H) 4.69 (br. s., 1 H) 7.09 (d, J=7.97 Hz, 1 H) 7.40 (d, J=7.97 Hz, 1 H)

### INTERMEDIATE 11

### 7-lodo-6-methyl-1,2-benzisoxazol-3-amine

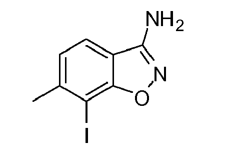

Potassium tert-butoxide (3.58 g, 31.87 mmol) was added to a stirred suspension of N-hydroxyacetamide (2.39 g, 31.87 mmol) in *N,N'*-dimethylformamide (44 mL) at room temperature under argon. After 30 minutes, 2-fluoro-3-iodo-4-methylbenzonitrile (Intermediate 10b, 3.33 g, 12.75 mmol) was added to the stirred reaction mixture at room temperature. After stirring overnight, the solvent was evaporated under reduced pressure, and the residue was partitioned between satured aqueous ammonium chloride solution and ethyl acetate. The organic layer was washed with brine, dried (MgS0₄) and evaporated. Purification of the residue by flash chromatography (4:1 hexanes/ethyl acetate to 1:1 hexanes/ethyl acetate) gave the title compound (2.21 g, 63%) as a pale yellow solid.
LRMS (m/z): 275 (M+1)⁺.
1H NMR (300 MHz, CHLOROFORM-*d*) δ ppm 2.59 (s, 3 H) 4.39 (br. s., 2 H) 7.15 (d, J=7.97 Hz, 1 H) 7.37 (d, J=7.97 Hz, 1 H)

### EXAMPLES

### EXAMPLE 1

### N-Cyclopropyl-3-fluoro-4-methyl-5-(3-oxo-2,3-dihydro[1,2,4]triazolo[4,3-a]pyridin-7-yl)benzamide

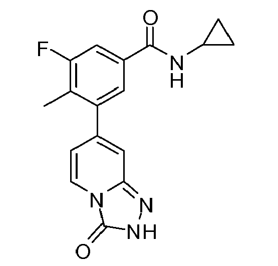

A Schlenk tube was charged with 7-iodo[1,2,4]triazolo[4,3-a]pyridin-3(2*H*)-one (Intermediate 1 b, 0.25 g, 0.75 mmol), *N*-cyclopropyl-3-fluoro-4-methyl-5-(4,4,5,5-tetramethyl-1,3,2-dioxaborolan-2-yl)benzamide (Intermediate 2b, 0.24 g, 0.76 mmol), cesium carbonate (2M aqueous solution, 1.20 mL, 2.40 mmol) and dioxane (8 mL). The mixture was submitted to three vacuum-argon cycles, then [1,1'-bis(diphenylphosphino)ferrocene]dichloropalladium(II) complex with dichloromethane (1:1) (0.06 g, 0.07 mmol) was added and the mixture purged in the same way. The reaction was stirred at 90° C under argon for 17 h. Subsequently, the reaction crude was filtered through Celite® washing with ethyl acetate. This organic phase was washed with water and brine, dried (Na₂SO₄) and concentrated. Purification of the residue by flash chromatography (9:1 ethyl acetate/ethanol) gave the title compound (0.08 g, 34%) as a solid.
LRMS (m/z): 327 (M+1)⁺.
1H NMR (300 MHz, DMSO-*d*6) δ ppm 0.56 - 0.65 (m, 2 H) 0.70 - 0.80 (m, 2 H) 2.27 (s, 3 H) 2.89 (ddd, J=7.62, 3.65, 3.33 Hz, 1 H) 6.67 (dd, J=7.30, 1.27 Hz, 1 H) 7.30 (d, J=0.95 Hz, 1 H) 7.63 - 7.77 (m, 2 H) 7.94 (d, J=7.30 Hz, 1 H) 8.60 (d, J=3.81 Hz, 1 H) 12.62 (br. s., 1 H)

### EXAMPLE 2

### N-cyclopropyl-3-[2-(2,2-dimethylpropyl)-3-oxo-2,3-dihydro[1,2,4]triazolo[4,3-a]pyridin-7-yl]-5-fluoro-4-methylbenzamide

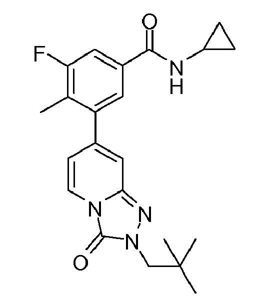

Obtained as a solid (29%) from 2-(2,2-dimethylpropyl)-7-iodo[1,2,4]triazolo[4,3-a]pyridin-3(2*H*)-one (Intermediate 3b) and N-cyclopropyl-3-fluoro-4-methyl-5-(4,4,5,5-tetramethyl-1,3,2-dioxaborolan-2-yl)benzamide (Intermediate 2b) following the experimental procedure as described in Example 1 and followed by purification of the crude product by reverse phase chromatography (C-18 silica column from Waters, using water/acetonitrile as eluents [0.1 % v/v formic acid buffered] 0% to 60%).
LRMS (m/z): 397 (M+1)⁺.
1H NMR (300 MHz, CHLOROFORM-*d*) δ ppm 0.58 - 0.67 (m, 2 H) 0.85 - 0.94 (m, 2 H) 1.07 (s, 9 H) 2.26 (d, J=2.47 Hz, 3 H) 2.85 - 2.97 (m, 1 H) 3.82 (s, 2 H) 6.25 (br. s., 1 H) 6.44 (dd, J=7.14, 1.37 Hz, 1 H) 7.01 (t, J=1.24 Hz, 1 H) 7.42 (s, 1 H) 7.48 (dd, J=9.61, 1.37 Hz, 1 H) 7.82 (dd, J=7.14, 1.10 Hz, 1 H)

### EXAMPLE 3

### tert-Butyl 4-[7-{5-[(cyclopropylamino)carbonyl]-3-fluoro-2-methylphenyl)-3-oxo[1,2,4]triazolo[4,3-a]pyridin-2(3H)-yl]piperidine-1-carboxylate

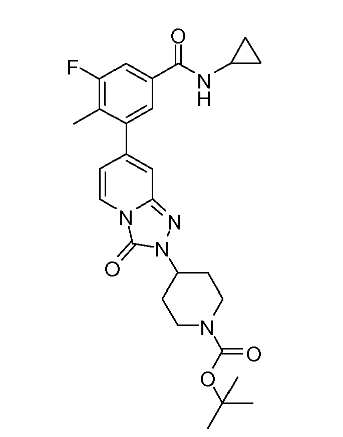

Obtained as a solid (78%) from *tert*-butyl 4-(7-iodo-3-oxo[1,2,4]triazolo[4,3-a]pyridin-2(3*H*)-yl)piperidine-1-carboxylate (Intermediate 4b) and N-cyclopropyl-3-fluoro-4-methyl-5-(4,4,5,5-tetramethyl-1,3,2-dioxaborolan-2-yl)benzamide (Intermediate 2b) following the experimental procedure as described in Example 1 and followed by purification of the crude product by flash chromatography (7:3 hexanes/ethyl acetate).
LRMS (m/z): 510 (M+1)⁺.
1H NMR (300 MHz, CHLOROFORM-*d*) δ ppm 0.56 - 0.71 (m, 2 H) 0.82 - 0.98 (m, 2 H) 1.49 (s, 9 H) 1.85 - 1.98 (m, 2 H) 1.98 - 2.15 (m, 3 H) 2.27 (d, J=2.47 Hz, 3 H) 2.82 - 3.01 (m, 3 H) 4.29 (br. s., 1 H) 4.43 - 4.58 (m, 1 H) 6.28 (br. s., 1 H) 6.46 (d, J=7.42 Hz, 1 H) 7.01 (s, 1 H) 7.43 (s, 1 H) 7.49 (d, *J*=11.26 Hz, 1 H) 7.83 (d, J=7.14 Hz, 1 H)

### EXAMPLE 4

### N-cyclopropyl-3-fluoro-4-methyl-5-(3-oxo-2-piperidin-4-yl-2,3-dihydro[1,2,4]triazolo[4,3-a]pyridin-7-yl)benzamide

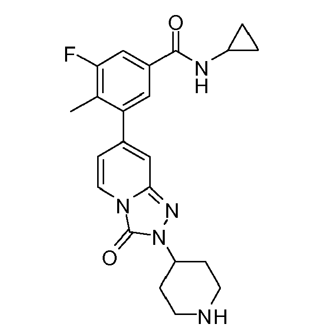

Hydrogen chloride 37% (4 mL) was added to a stirred solution of *tert-butyl* 4-[7-{f5-[(cyclopropylamino)carbonyl]-3-fluoro-2-methylphenyl}-3-oxo[1,2,4]triazolo[4,3-a]pyridin-2(3*H*)-yl]piperidine-1-carboxylate (Example 3, 0.21 g, 0.41 mmol) in tetrahydrofuran (4 mL) and the mixture was stirred at room temperature. After 2 hours, satured aqueous sodium carbonate solution was added and the mixture was extracted further with dichloromethane. The organic layer was dried (MgSO₄) and the solvent was removed under vacuum. The crude was purified by reverse phase chromatography (C-18 silica column from Waters, water/acetonitrile as eluents [and 1% of ammonia] 0% to 60%) to give the title compound (0.15 g, 92%) as a solid.
LRMS (m/z): 410 (M+1)⁺.
1H NMR (300 MHz, CHLOROFORM-*d*) δ ppm 0.58 - 0.68 (m, 2 H) 0.84 - 0.94 (m, 2 H) 1.66 - 1.79 (m, 1 H) 1.83 - 1.93 (m, 1 H) 1.95 - 2.22 (m, 4 H) 2.26 (s, 3 H) 2.79 - 2.97 (m, 2 H) 3.34 (d, J=15.93 Hz, 2 H) 4.42 - 4.55 (m, 1 H) 6.37 (br. s., 1 H) 6.45 (dd, J=7.14, 1.37 Hz, 1 H) 7.02 (s, 1 H) 7.43 (s, 1 H) 7.49 (dd, J=9.61, 1.65 Hz, 1 H) 7.81 (d, J=8.24 Hz, 1 H)

### EXAMPLE 5

### N-cyclopropyl-3-fluoro-4-methyl-5-{2-[1-(methylsulfonyl)piperidin-4-yi]-3-oxo-2,3-dihydro[1,2,4]triazolo[4,3-a]pyridin-7-yl}benzamide

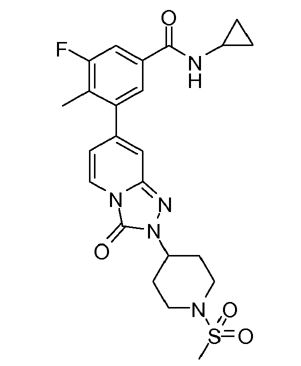

Methanesulfonyl chloride (0.08 mL) was added to a stirred solution of N-cyclopropyl-3-fluoro-4-methyl-5-(3-oxo-2-piperidin-4-yl-2,3-dihydro[1,2,4]triazolo[4,3-a]pyridin-7-yl)benzamide (Example 4, 0.13 g, 0.32 mmol) and triethylamine (0.18 mL, 1.29 mmol) in dichloromethane (3 mL) and the mixture was stirred at room temperature. After 1 hour, saturated aqueous ammonium chloride solution and water were added and the mixture was extracted with additional dichloromethane. The organic layer was dried (MgSO₄) and the solvent was removed under vacuum. The crude was purified by flash chromatography (10:1 ethyl acetate/ethanol) to give the title compound (0.12 g, 79%) as a solid.
LRMS (m/z): 488 (M+1)⁺.
1H NMR (300 MHz, CHLOROFORM-*d*) δ ppm 0.55 - 0.72 (m, 2 H) 0.81 - 0.98 (m, 2 H) 1.17 - 1.35 (m, 1 H) 1.64 (br. s., 2 H) 2.07 (d, J=12.91 Hz, 2 H) 2.26 (s, 3 H) 2.86 (s, 3 H) 2.89 - 3.06 (m, 1 H) 3.97 (d, *J*=11.81 Hz, 2 H) 4.12 (br. s., 1 H) 4.32 - 4.61 (m, 1 H) 6.28 (br. s., 1 H) 6.48 (d, J=3.02 Hz, 1 H) 7.27 (br. s., 1 H) 7.37 - 7.59 (m, 2 H) 7.82 (d, J=2.75 Hz, 1 H)

### EXAMPLE 6

### N-Cyclopropyl-3-fluoro-4-methyl-5-[3-oxo-2-(tetrahydro-2H-pyran-4-yl)-2,3-dihydro[1,2,4]triazolo[4,3-a]pyridin-7-yl]benzamide

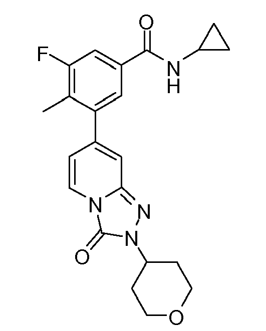

Obtained as a solid (49%) from 7-iodo-2-(tetrahydro-2H-pyran-4-yl)[1,2,4]triazolo[4,3-a]pyridin-3(2*H*)-one (Intermediate 5b) and N-cyclopropyl-3-fluoro-4-methyl-5-(4,4,5,5-tetramethyl-1 ,3,2-dioxaborolan-2-yl)benzamide (Intermediate 2b) following the experimental procedure as described in Example 1 and followed by purification of the crude product by flash chromatography (7:3 hexanes/ethyl acetate).
LRMS (m/z): 411 (M+1)⁺.
1H NMR (300 MHz, CHLOROFORM-*d*) δ ppm 0.65 (d, J=1.37 Hz, 2 H) 0.83 - 0.99 (m, 2 H) 1.91 (d, J=12.36 Hz, 2 H) 2.14 - 2.26 (m, 2 H) 2.28 (s, 3 H) 2.92 (ddd, J=6.94, 3.36, 3.16 Hz, 1 H) 3.60 (t, *J*=11.95 Hz, 2 H) 4.15 (d, J=8.51 Hz, 2 H) 4.51 - 4.67 (m, 1 H) 6.33 (br. s., 1 H) 6.43 - 6.52 (m, 1 H) 7.04 (d, J=1.37 Hz, 1 H) 7.45 (s, 1 H) 7.51 (d, *J*=9.61 Hz, 1 H) 7.84 (d, J=7.14 Hz, 1 H)

### EXAMPLE 7

### 3-[2-(2-Chlorophenyl)-3-oxo-2,3-dihydro[1,2,4]triazolo[4,3-a]pyridin-7-yl]-N-cyclopropyl-5-fluoro-4-methylbenzamide

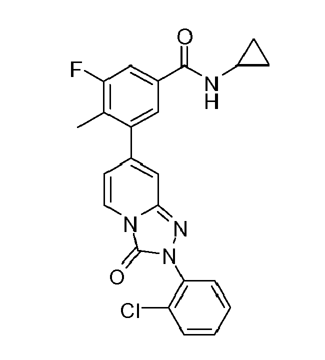

A mixture of *N*-cyclopropyl-3-fluoro-4-methyl-5-(3-oxo-2,3-dihydro[1,2,4]triazolo[4,3-a]pyridin-7-yl)benzamide (Example 1, 0.17 g, 0.51 mmol), 1-chloro-2-iodobenzene (0.08 mL, 0.63 mmol), potassium carbonate (0.15 g, 1.08 mmol), *trans-N,N'-*dimethylcyclohexane-1,2-diamine (0.02 g, 0.10 mmol) and dimethylsulphoxide (5 mL) was submitted to three vacuum-argon cycles, then iodocopper (0.01 g, 0.05 mmol) was added and the mixture purged in the same way. The mixture was heated at 160 °C for 6 hours in a Biotage Initiator Microwave Synthesizer. The mixture was then cooled and ethyl acetate was added. The organic layer was washed with water, dried (MgSO₄) and evaporated. Purification of the residue by flash chromatography (8:2 hexanes/ethyl acetate to 1:1 hexanes/ethyl acetate) gave the title compound (0.04 g, 21 %) as a solid.
LRMS (m/z): 437 (M+1)⁺.
1H NMR (300 MHz, CHLOROFORM-*d*) δ ppm 0.58 - 0.70 (m, 2 H) 0.82 - 0.97 (m, 2 H) 2.30 (s, 3 H) 2.86 - 2.96 (m, 1 H) 6.24 (br. s., 1 H) 6.51 (d, J=7.42 Hz, 1 H) 7.06 (s, 1 H) 7.39 - 7.53 (m, 4 H) 7.54 - 7.66 (m, 2 H) 7.90 (d, J=7.42 Hz, 1 H)

### EXAMPLE 8

### 2-(2,2-dimethylpropyl)-7-[2-methyl-5-(5-methyl-1,3,4-thiadiazol-2-yl)phenyl][1,2,4]triazolo[4,3-a]pyridin-3(2l-n-one

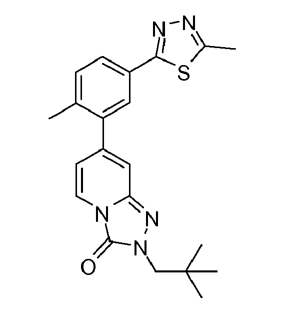

Obtained as a solid (37%) from 2-(3-iodo-4-methylphenyl)-5-methyl-1,3,4-thiadiazole (Intermediate 6b) and 2-(2,2-dimethylpropyl)-7-(4,4,5,5-tetramethyl-1,3,2-dioxaborolan-2-yl)[1,2,4]triazolo[4,3-a]pyridin-3(2*H*)-one (Intermediate 7) following the experimental procedure as described in Example 1 and followed by purification of the crude product by flash chromatography (6:4 hexanes/ethyl acetate to 2:8 hexanes/ethyl acetate).
LRMS (m/z): 394 (M+1)⁺.
1H NMR (300 MHz, CHLOROFORM-*d*) δ ppm 1.08 (s, 9 H) 2.39 (s, 3 H) 2.83 (s, 3 H) 3.83 (s, 2 H) 6.51 (dd, J=7.28, 1.51 Hz, 1 H) 7.06 (d, J=1.10 Hz, 1 H) 7.40 (d, J=7.97 Hz, 1 H) 7.78 - 7.91 (m, 3 H)

### EXAMPLE 9

### 2-(2,2-dimethylpropyl)-7-[2-methyl-5-(5-methyl-1,3,4-oxadiazol-2-yl)phenyl][1,2,4]triazolo[4,3-a]pyridin-3(2H)-one

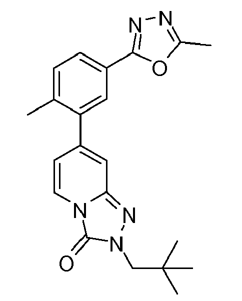

Obtained as a solid (45%) from 2-(2,2-dimethylpropyl)-7-iodo[1,2,4]triazolo[4,3-a]pyridin-3(2*H*)-one (Intermediate 3b) and [2-methyl-5-(5-methyl-1,3,4-oxadiazol-2-yl)phenyl]boronic acid (Intermediate 8d) following the experimental procedure as described in Example 1 and followed by purification of the crude product by flash chromatography (1:1 hexanes/ethyl acetate).
LRMS (m/z): 378 (M+1)⁺.
1H NMR (300 MHz, CHLOROFORM-*d*) δ ppm 1.09 (s, 9 H) 2.41 (s, 3 H) 2.63 (s, 3 H) 3.84 (s, 2 H) 6.52 (dd, J=7.28, 2.06 Hz, 1 H) 7.06 (s, 1 H) 7.45 (d, J=7.97 Hz, 1 H) 7.84 (d, J=7.97 Hz, 1 H) 7.93 (d, J=1.65 Hz, 1 H) 7.99 (dd, J=7.97, 1.65 Hz, 1 H)

### EXAMPLE 10

### 2-(2,2-dimethylpropyl)-7-[3-fluoro-2-methyl-5-(5-methyl-4H-1,2,4-triazol-3-yl)phenyl][1,2,4]triazolo[4,3-a]pyridin-3(2l-n-one

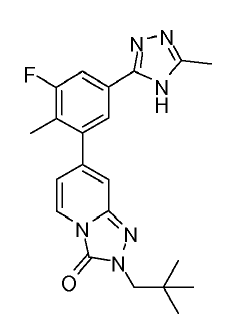

Obtained as a solid (20%) from 3-(3-fluoro-5-iodo-4-methylphenyl)-5-methyl-4H-1,2,4-triazole (Intermediate 9d) and 2-(2,2-dimethylpropyl)-7-(4,4,5,5-tetramethyl-1,3,2-dioxaborolan-2-yl)[1,2,4]triazolo[4,3-a]pyridin-3(2*H*)-one (Intermediate 7) following the experimental procedure as described in Example 1 and followed by purification of the crude product by flash chromatography (6:4 hexanes/ethyl acetate to 100% ethyl acetate).
LRMS (m/z): 395 (M+1)⁺.
1H NMR (300 MHz, CHLOROFORM-*d*) δ ppm 1.09 (s, 9 H) 2.29 (d, J=2.20 Hz, 3 H) 2.57 (s, 3 H) 3.84 (s, 2 H) 6.53 (dd, J=7.28, 1.51 Hz, 1 H) 7.08 (d, J=1.10 Hz, 1 H) 7.25 - 7.28 (m, 1 H) 7.73 - 7.89 (m, 2 H)

### EXAMPLE 11

### 7-[3-(cyclopropylamino)-6-methyl-1,2-benzisoxazol-7-yl]-2-(2,2-dimethylpropyl)[1,2,4]triazolo[1,2,4]pyridin-3(2H)-one

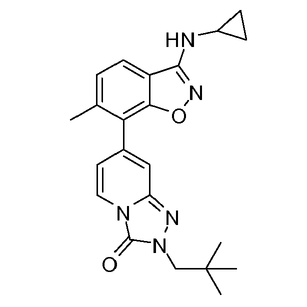

Obtained as a solid (21%) from *N*-cyclopropyl-7-iodo-6-methyl-1,2-benzisoxazol-3-amine (Intermediate 10h) and 2-(2,2-dimethylpropyl)-7-(4,4,5,5-tetramethyl-1,3,2-dioxaborolan-2-yl)[1,2,4]triazolo[4,3-a]pyridin-3(2*H*)-one (Intermediate 7) following the experimental procedure as described in Example 1 and followed by purification of the crude product by flash chromatography (6:4 hexanes/ethyl acetate to 2:8 hexanes/ethyl acetate).
LRMS (m/z): 392 (M+1)⁺.
1H NMR (300 MHz, CHLOROFORM-*d*) δ ppm 0.66 - 0.77 (m, 2 H) 0.81 - 0.93 (m, 2 H) 1.07 (s, 9 H) 2.45 (s, 3 H) 2.77 - 2.91 (m, J=5.77, 5.42, 5.42, 3.71 Hz, 1 H) 3.83 (s, 2 H) 4.72 (br. s., 1 H) 6.58 (d, J=6.87 Hz, 1 H) 7.12 - 7.22 (m, 2 H) 7.49 (d, J=7.97 Hz, 1 H) 7.84 (d, J=7.14 Hz, 1 H)

### EXAMPLE 12

### 7-(3-amino-6-methyl-1,2-benzisoxazol-7-yl)-2-(2,2-dimethylpropyl)[1,2,4]triazolo[4,3-a]pyridin-3(2H)-one

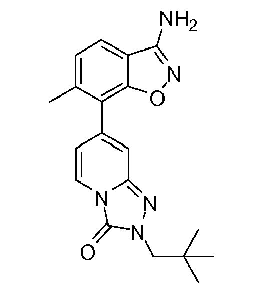

Obtained as a beige solid (18%) from 7-iodo-6-methyl-1,2-benzisoxazol-3-amine (Intermediate 11) and 2-(2,2-dimethylpropyl)-7-(4,4,5,5-tetramethyl-1,3,2-dioxaborolan-2-yl)[1,2,4]triazolo[4,3-a]pyridin-3(2*H*)-one (Intermediate 7) following the experimental procedure as described in Example 1 and followed by purification of the crude product by reverse phase chromatography (C-18 silica column from Waters, water/acetonitrile as eluents [0.1% v/v formic acid buffered] 20% to 60%).
LRMS (m/z): 352 (M+1)⁺.
1H NMR (300 MHz, CHLOROFORM-*d*) δ ppm 1.08 (s, 9 H) 2.46 (s, 3 H) 3.83 (s, 2 H) 4.40 (br. s., 2 H) 6.57 (d, J=6.59 Hz, 1 H) 7.19 (d, J=12.09 Hz, 1 H) 7.27 (d, J=0.55 Hz, 1 H) 7.46 (d, J=8.51 Hz, 1 H) 7.86 (d, J=7.14 Hz, 1 H)

## Claims

1. A compound of formula (l) or a pharmaceutically acceptable salt or N-oxide thereof 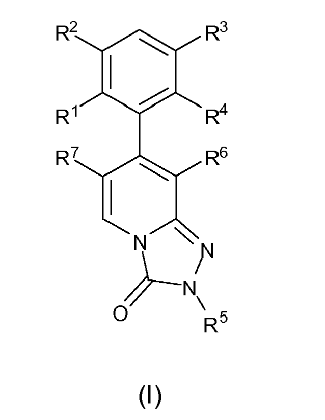 wherein
• R¹ and R² are independently selected from the group consisting of a hydrogen atom, a halogen atom and a C₁₋₃ alkyl group;
• either (a) R³ is selected from the group consisting of a -CONR^{a}R^{b} group and a 5 to 7-membered heteroaryl group, wherein said heteroaryl group is optionally substituted with a C₁₋₃ alkyl group; and R⁴ is selected from the group consisting of a hydrogen atom, a halogen atom and a C₁₋₃ alkyl group; or (b) R³ together with R⁴ forms a 5 to 7-membered heteroaryl group, wherein said heteroaryl group is optionally substituted with one or more substituents selected from an amino group, a C₁₋₃ alkylamino group and a C₃₋₇ cycloalkylamino;
• R⁵ is selected from the group consisting of a hydrogen atom, a C₁₋₆ alkyl group, a C₆₋₁₀ aryl group, a 5 to 7-membered heteroaryl group, a C₃₋₇ cycloalkyl group and a 3 to 7-membered heterocyclyl group, wherein said aryl, heteroaryl, cycloalkyl and heterocyclyl groups are optionally substituted with one or more substituents selected from a halogen atom, a C₁₋₃ alkyl group, a C₁₋₃ alkylsulfonyl group and a C₁₋₄ alkoxycarbonyl group;
• R⁶ and R⁷ are independently selected from the group consisting of a hydrogen atom, a halogen atom and a C₁₋₃ alkyl group,
• R^{a} and R^{b} are independently selected from the group consisting of a hydrogen atom, a C₁₋₆ alkyl group and a C₃₋₇ cycloalkyl group; or R^{a} and R^{b} together with the nitrogen atom to which they are attached form a 3 to 7-membered heterocyclyl group;
with the proviso that when R¹ is a hydrogen atom, one of R⁶ or R⁷ is other than a hydrogen atom.

2. A compound according to claim 1 wherein R⁷ represents a C₁₋₃ alkyl group, preferably R⁷ represents a methyl group.

3. A compound according to claim 1 or 2 wherein R² represents a halogen atom, preferably R² represents a fluorine atom.

4. A compound according to any one of claims 1 to 3 wherein R³ represents a -CONR^{a}R^{b} group or R³ together with R⁴ forms a 5 to 7-membered heteroaryl group optionally substituted with an amino group or a C₃₋₇ cycloalkylamino group, preferably R³ represents a -CONR^{a}R^{b} group.

5. A compound according to any one of claims 1 to 3 wherein R⁴ is a hydrogen atom or together with R³ form a 5 to 7-membered heteroaryl group optionally substituted with an amino group or a C₃₋₇ cycloalkylamino group, preferably R⁴ represents a hydrogen atom.

6. A compound according to any one of claims 1 to 5 wherein R⁵ represents a C₁₋₆ alkyl group or a phenyl group optionally substituted with one or two halogen atoms.

7. A compound according to any one of claims 1 to 6 wherein R⁶ and R⁷ independently represent a hydrogen atom.

8. A compound according to claims 1 to 7 wherein:
• R¹ represents a methyl group;
• R² represents a fluorine atom;
• R³ represents a -CONR^{a}R^{b} group;
• R⁴ represents a hydrogen atom; or R³ together with R⁴ form a 5 to 7-membered heteroaryl group optionally substituted with an amino group or a C₃₋₇ cycloalkylamino group;
• R⁵ represents a C₁₋₆ alkyl group or a phenyl group optionally substituted by one or two halogen atoms; and
• R⁶ and R⁷ independently represent a hydrogen atom.

9. A compound according to claim 1 wherein:
• R¹ represents a methyl group;
• R² represents a hydrogen atom or a fluorine atom;
• R³ represents a 5-methyl-1,3,4-thiadiazol-2-yl group, a 5-methyl-1,3,4-oxadiazol-2-yl group, a 5-methyl-4H-1,2,4-triazol-3-yl group or a cyclopropylcarbamoyl group;
• R⁴ represents a hydrogen atom or R³ together with R⁴ form an isoxazole ring, wherein said ring is substituted with an amino group or a cyclopropylamino group;
• R⁵ represents a hydrogen atom, a neopentyl group, a tetrahydro-2H-pyran-4-yl group, a piperidinyl-4-group, a *N-*(*tert*-butoxycarbonyl)piperidin-4-yl group, a N-(methylsulfonyl)piperidin-4-yl group, or a phenyl group substituted with a chlorine atom; and
• R⁶ and R⁷ represent a hydrogen atom.

10. A compound according to claim 1 which is one of:
• N-Cyclopropyl-3-fluoro-4-methyl-5-(3-oxo-2,3-dihydro[1,2,4]triazolo[4,3-*a*]pyridin-7-yl)benzamide;
• N-cyclopropyl-3-[2-(2,2-dimethylpropyl)-3-oxo-2,3-dihydro[1,2,4]triazolo[4,3-a]pyridin-7-yl]-5-fluoro-4-methylbenzamide;
• *tert*-Butyl4-[7-f5-[(cyclopropylamino)carbonyl]-3-fluoro-2-methylphenyll-3-oxo-[1,2,4]triazolo[4,3-a]pyridin-2(3H)-yl]piperidine-1-carboxylate;
• *N*-cyclopropyl-3-fluoro-4-methyl-5-(3-oxo-2-piperidin-4-yl-2,3-dihydro[1,2,4]-triazolo[4,3-a]pyridin-7-yl)benzamide;
• *N*-cyclopropyl-3-fluoro-4-methyl-5-f2-[l -(methylsulfonyl)piperidin-4-yl]-3-oxo-2,3-dihydro[1 ,2,4]triazolo[4,3-a]pyridin-7 -yl}benzamide;
• N-Cyclopropyl-3-fluoro-4-methyl-5-[3-oxo-2-(tetrahydro-*2H*-pyran-4-yl)-2,3-dihydro[1,2,4]triazolo[4,3-a]pyridin-7-yl]benzamide;
• 3-[2-(2-Chlorophenyl)-3-oxo-2,3-dihydro[1,2,4]triazolo[4,3-a]pyridin-7-yl]-*N-*cyclopropyl-5-fluoro-4-methylbenzamide;
• 2-(2,2-dimethylpropyl)-7-[2-methyl-5-(5-methyl-1,3,4-thiadiazol-2-yl)phenyl][1,2,4]-triazolo[4,3-*a*]pyridin-3(2H)-one;
• 2-(2,2-dimethylpropyl)-7-[2-methyl-5-(5-methyl-1,3,4-oxadiazol-2-yl)phenyl][1,2,4]-triazolo[4,3-a]pyridin-3(2*H*)-one;
• 2-(2,2-dimethylpropyl)-7-[3-fluoro-2-methyl-5-(5-methyl-4H-1,2,4-triazol-3-yl)phenyl][1,2,4]triazolo[4,3-*a*]pyridin-3(*2H*)-one;
• 7-[3-(cyclopropylamino)-6-methyl-1,2-benzisoxazol-7-yl]-2-(2,2-dimethylpropyl)-[1,2,4]triazolo[4,3-*a*]pyridin-3(2H)-one; or
• 7-(3-amino-6-methyl-1,2-benzisoxazol-7-yl)-2-(2,2-dimethylpropyl)[1,2,4]triazolo[4,3-*a*]pyridin-3(*2H*)-one.

11. A compound according to any one of claims 1 to 10 for use in the treatment of a pathological condition or disease susceptible to amelioration by inhibition of the p38 mitogen-activated protein kinase.

12. A compound according to claim 11, wherein the pathological condition or disease is rheumatoid arthritis, ischemia-reperfusion injury, cerebral focal ischemia, acute coronary syndrome, asthma, COPD, Crohn's disease, irritable bowel syndrome, adult respiratory distress syndrome, osteoporosis, Alzheimer's disease, rheumatoid spondylitis, psoriasis, atherosclerosis, osteoarthritis or multiple myeloma.

13. A pharmaceutical composition comprising a compound as defined in any one of claims 1 to 10 in admixture with a pharmaceutically acceptable diluent or carrier.

14. Use of a compound as defined in any one of claims 1 to 10 in the manufacture of a medicament for the treatment of a pathological condition or disease as defined in claims 11 or 12.

15. A method for treating a subject afflicted with a pathological condition or disease as defined in claims 11 or 12, which comprises administering to said subject an effective amount of a compound as defined in any one of claims 1 to 10.

16. A combination product comprising:
(i) a compound according to any one of claims 1 to 10; and
(ii) one or more compounds selected from antagonists of M3 muscarinic receptors, β2-agonists, PDE4 inhibitors, cortiocosteroids, leukotriene D4 antagonists, inhibitors of Egfr-kinase, antagonists of the A2B adenosine receptor, Pl3Kδγ inhibitors, NK1 receptor agonists, CRTh2 antagonists, Syk kinase inhibitors, CCR3 antagonists, VLA-4 antagonists and DMARDs (disease modifying antirheumatic drugs)
for simultaneous, separate or sequential use in the treatment of the human or animal body.
